(19) Europäisches Patentamt
European Patent Office
Office européen des brevets

(11) Publication number: 0 242 169 B1

# (12) EUROPEAN PATENT SPECIFICATION

(45) Date of publication of patent specification: 02.01.92

(51) Int. Cl.[5]: C07D 307/88, C07D 405/14, C07D 405/06, C07D 471/04, B41M 5/00, //(C07D471/04, 221:00,221:00)

(21) Application number: 87303230.4

(22) Date of filing: 13.04.87

## (54) Divinyl compounds and chromogenic recording material prepared by use thereof.

(30) Priority: 15.04.86 JP 87619/86
02.02.87 JP 23361/87

(43) Date of publication of application:
21.10.87 Bulletin 87/43

(45) Publication of the grant of the patent:
02.01.92 Bulletin 92/01

(84) Designated Contracting States:
BE CH DE FR GB IT LI

(56) References cited:
EP-A- 0 062 544
EP-A- 0 188 377
DE-A- 2 218 895
DE-A- 2 614 944

CHEMICAL ABSTRACTS, vol. 102, no. 22, 3rd June 1985, page 684, abstract no. 195296f, Columbus, Ohio, US; & JP - A - 60 27589

(73) Proprietor: YAMADA CHEMICAL CO., LTD.
1-1 Kamitoba-Kamichoshicho Minami-ku
Kyoto-shi Kyoto-fu(JP)

(72) Inventor: Kawai, Hajime
3-12-12 Osumigaoka Tanabe-cho
Tsuzuki-gun Kyoto-fu(JP)
Inventor: Fujino, Yoshiharu
3-14-23 Osumigaoka Tanabe-cho
Tsuzuki-gun Kyoto-fu(JP)
Inventor: Shimizu, Youji
54 Daigo-Kakiharacho Fushimi-ku
Kyoto-shi Kyoto-fu(JP)
Inventor: Nieda, Seiichi 205 Rumieru-Nishikyogoku
6-Nishikyogoku-Suehiro-cho
Ukyo-ku Kyoto-shi Kyoto-fu(JP)
Inventor: Gendai, Kazuhiko 6-1-1106
Mukaijima-Danchi
14-8 Mukaijima-Yotsuyaike
Fushimi-ku Kyoto-shi Kyoto-fu(JP)

EP 0 242 169 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**CHEMICAL ABSTRACTS, vol. 102, no. 26, 1st July 1985, page 589, abstract no. 229544c, Columbus, Ohio, US; & JP - A - 60 08364**

**CHEMICAL ABSTRACT, vol. 107, no. 6, 10th August 1987, page 663, abstract no. 49640a, Columbus, Ohio, US; & JP - A - 61 202 883**

Inventor: **Tsunemitsu, Katsuhiko**
**30-3 Daigo-Takahatacho Fushimi-ku**
**Kyoto-shi Kyoto-fu(JP)**

(74) Representative: **Woods, Geoffrey Corlett et al**
**J.A. KEMP & CO. 14 South Square Gray's Inn**
**London WC1R 5EU(GB)**

## Description

The present invention relates to a divinyl compound suitable for use as a chromogenic agent in a recording-material such as a pressure-sensitive recording paper, heat-sensitive recording paper or electro-heat sensitive recording paper, to a process for the preparation of such a compound and to a recording material incorporating such a compound.

The color image provided by the following black-colouring fluorane compound (A), which has been used as a conventional chromogenic agent for a recording-material, does not have any absorption in the near infrared region. Accordingly the color image cannot be read by an optical letter-reading apparatus (refer to Figure 2):

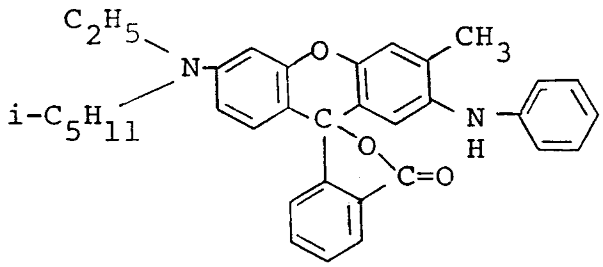

(A)

(refer to JP-B-56-52759/1081).

Chromogenic agents having an absorption in the near infrared region have been recently proposed in JP-B-58-5940/1983, JP-A-59-199757/1984 and JP-A-60-230890/1985. However, each of the proposed compounds has the following defects and a satisfactory chromogenic agent has not yet been obtained.

Compound (B) (see below) of JP-B-58-5940/1983 and compound (C) (see below) of JP-A-60-230890/1985 are themselves strongly colored yellow and besides spontaneously color. These defects have a very bad effect on the production of recording materials.

Although the fluorene compound (D) (see below) of JP-A-59-199757/1984 is colorless, its chromogenic property and the stability of color image produced are poor.

Furthermore, the hue of each of compounds (B), (C) and (D) when developed is green. Accordingly, to obtain a blackish color, another chromogenic agent giving a red or black colour must be added in a large amount. Since the chromogenic property and the chromogenic speed of such an agent are different from those of compounds (B), (C) and (D) and particularly, the light-resistance of red-chromogenic agents is generally poor, adverse influences such as unbalance of color-development and reduction of light-resistance cannot be avoided.

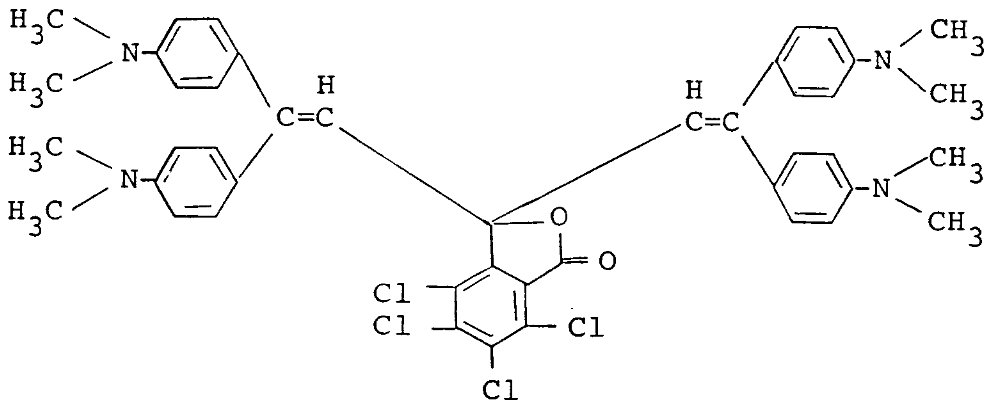

(B)

(refer to JP-B-58-5940/1983).

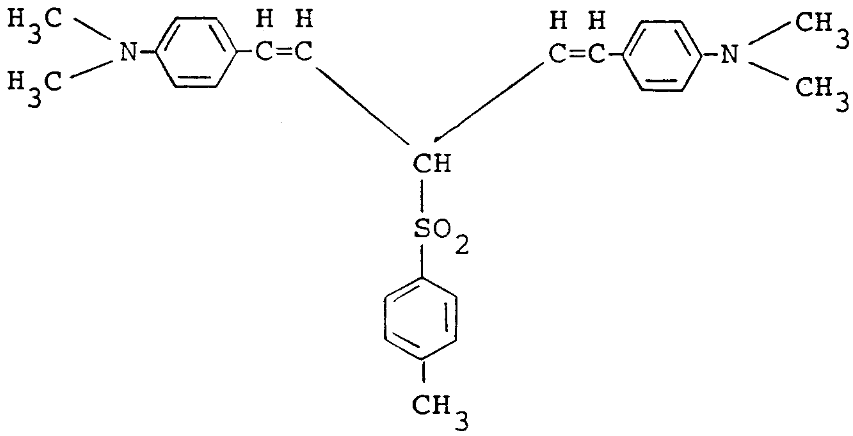

(C)

(refer to JP-A-60-230890/1985).

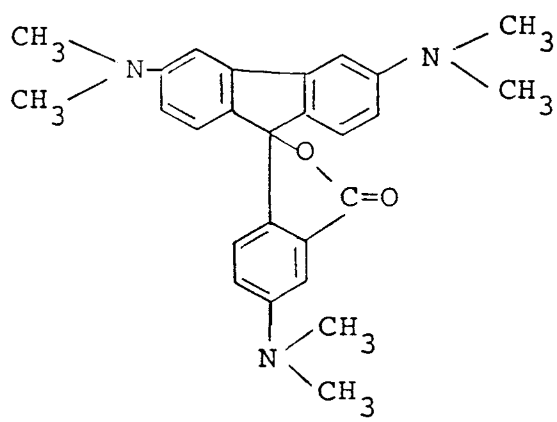

(D)

(refer to JP-A-59-199757/1984).

The present inventors have sought to improve the defects of the conventional chromogenic agents.

The present invention provides a divinyl phthalide compound represented by the formula (I):

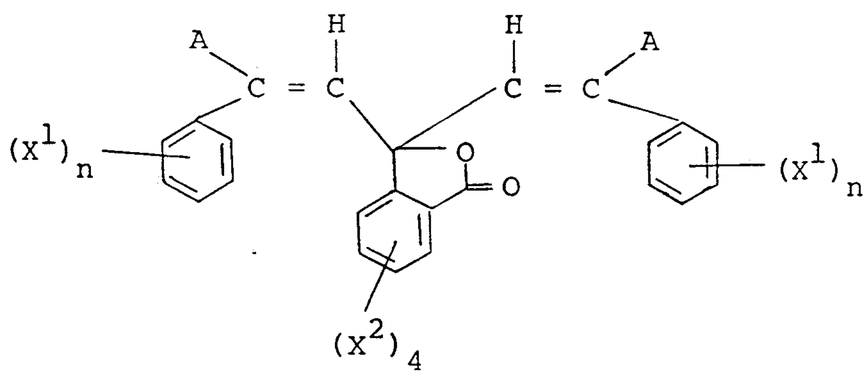

(I)

wherein A represents

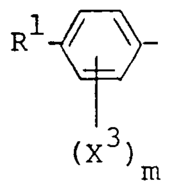 , 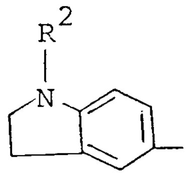 , 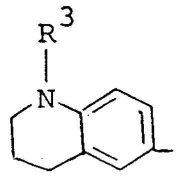 or 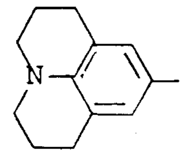 ,

$X^1$ represents an alkyl group, an alkoxy group, an alkoxy-alkoxy group, an aryloxy group, a cycloalkoxy group, a haloalkoxy group, an alkenyloxy group, an aralkyloxy group or a halogen atom;
$X^2$ represents a halogen atom;
$X^3$ represents an alkyl group having not more than eight carbon atoms, an alkoxy group having not more than eight carbon atoms or a halogen atom;
$R^1$ represents a heterocyclic ring having one or more nitrogen atoms;
$R^2$ and $R^3$ each represents a hydrogen atom, an alkyl group, an alkoxyalkyl group or a haloalkyl group; and m and n each represents 0, 1, 2 or 3; wherein each $X^1$ or each $X^3$ is the same or different when n or m respectively is 2 or 3 and each $X^2$ is the same or different.
(Hereinafter the same sign means the same meaning).

The divinyl compounds represented by the formula (I) are by themselves almost colorless, extremely stable in the atmosphere, have no subliming property and spontaneously chromogenic property and dissolve extremely well in organic solvents. They give a blackish colour rapidly with a developer and the color image produced has excellent light-resistance and moisture-resistance. Furthermore, since the color image has a strong absorption between 700 and 1000 nm in addition to the visible region, the color image has the distinctive feature that it is possible to be read by optical letter-reading apparatus using near infrared rays (such as OCR and OMR) and by barcode reading apparatus. Thus the divinyl compounds of the present invention (hereinafter sometimes referred to as the present compounds) are extremely valuable and can be used as chromogenic agents for ordinary recording-material requiring a black color, for which demand is rapidly increasing recently, as well as material readable by, for example, OCR or OMR.

Preferred compounds of the present invention are those wherein $R^1$ is

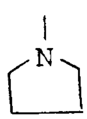 , 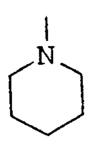 , 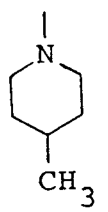 , 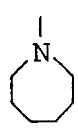 , 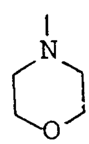 or

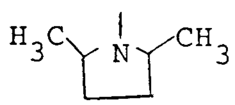 ;

$R^2$ is an alkyl group having not more than six carbon atoms; $R^3$ is an alkyl group having not more than six carbon atoms; $X^1$ is an alkyl group having not more than six carbon atoms, an alkoxy group having not more than six carbon atoms, a cyclohexyloxy group, a cyclopentyloxy group, a benzyloxy group, an allyloxy group, an alkoxyalkoxy group having not more than 6 carbon atoms in total, a phenoxy group, a phenoxy group substituted by an alkyl group or an alkoxy group or a halogen atom; $(X^2)_4$ represents four chlorine atoms, four bromine atoms, one chlorine atom and three bromine atoms or two chlorine atoms and two bromine atoms; $X^3$ is a methoxy group or an ethoxy group; m is 0 or 1 and n is 0, 1 or 2.

More preferred compounds are those wherein $R^1$ is

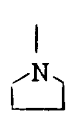

and m is 0, especially those wherein n is 1 and $X^1$ is an alkoxy group of not more than four carbon atoms in a para position, or those wherein A is

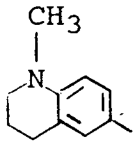

,

X is a p-methoxy group and n is 1.

The divinyl compounds represented by the formula (I) have unexpectedly excellent properties such as solubility, the color of the compound itself, the hue of the developed color, the chromogenic property, the absorbance of near infrared rays and the stability of the color image produced.

Of the attached drawings, Figures 1 and 2 are the reflection spectra of a color images of a pressure-sensitive and heat-sensitive recording paper incorporating the present compound and a comparison compound respectively, and Figure 3 is the reflection spectra of a texture of a heat-sensitive recording paper incorporating the present compound and the comparison compound.

Examples of the present compounds are given below. Every compound is an almost colorless solid and develops a blue-black to black color rapidly by the action of activated clay.

1. 3,3-Bis[2-(p-pyrrolidinophenyl)-2-(p-methoxyphenyl)ethenyl]-4,5,6,7-tetrachlorophthalide,
2. 3,3-Bis[2-(p-pyrrolidinophenyl)-2-(p-ethoxyphenyl)ethenyl]-4,5,6,7-tetrachlorophthalide,
3. 3,3-Bis[2-(p-pyrrolidinophenyl)-2-(p-propoxyphenyl)ethenyl]-4,5,6,7-tetrachlorophthalide,
4. 3,3-Bis[2-(p-pyrrolidinophenyl)-2-(p-iso-propoxyphenyl)ethenyl]-4,5,6,7-tetrachlorophthalide,
5. 3,3-Bis[2-(p-pyrrolidinophenyl)-2-(p-butoxyphenyl)ethenyl]4,5,6,7-tetrachlorophthalide,
6. 3,3-Bis[2-(p-pyrrolidinophenyl)-2-(p-iso-butoxyphenyl)ethenyl]-4,5,6,7-tetrachlorophthalide,
7. 3,3-Bis[2-(p-pyrrolidinophenyl)-2-(p-sec-butoxyphenyl)ethenyl]4,5,6,7-tetrachlorophthalide,
8. 3,3-Bis[2-(p-pyrrolidinophenyl)-2-(p-tert-butoxyphenyl)ethenyl]4,5,6,7-tetrachlorophthalide,
9. 3,3-Bis[2-(p-pyrrolidinophenyl)-2-(m-methyl-p-methoxyphenyl)ethenyl]4,5,6,7-tetrachlorophthalide,
l0. 3,3-Bis[2-(p-pyrrolidinophenyl)-2-(3,4-dimethoxyphenyl)ethenyl]-4,5,6,7-tetrachlorophthalide,
ll. 3,3-Bis[2-(p-pyrrolidinophenyl)-2-phenyl-ethenyl]4,5,6,7-tetrachlorophthalide,
l2. 3,3-Bis[2-(p-pyrrolidinophenyl)-2-(p-methylphenyl)ethenyl]-4,5,6,7-tetrachlorophthalide,
l3. 3,3-Bis[2-(p-pyrrolidinophenyl)-2-(p-methoxyphenyl)ethenyl]-4,5,6,7-tetrabromophthalide,
l4. 3,3-Bis[2-(p-pyrrolidinophenyl)-2-(p-pentyloxyphenyl)ethenyl]-4,5,6,7-tetrachlorophthalide,
l5. 3,3-Bis[2-(p-pyrrolidinophenyl)-2-(p-iso-pentyloxyphenyl)ethenyl]-4,5,6,7-tetrachlorophthalide,
l6. 3,3-Bis[2-(p-pyrrolidinophenyl)-2-(p-chlorophenyl)ethenyl]-4,5,6,7-tetrachlorophthalide,
l7. 3,3-Bis[2-(p-pyrrolidinophenyl)-2-(p-ethoxyphenyl)ethenyl]-4,5,6,7-tetrabromophthalide,
l8. 3,3-Bis[2-(p-pyrrolidinophenyl)-2-(m-methoxy-p-ethoxyphenyl) ethenyl]-4,5,6,7-tetrachlorophthalide,
l9. 3,3-Bis[2-(p-pyrrolidinophenyl)-2-(m-methyl-p-ethoxyphenyl)ethenyl]-4,5,6,7-tetrachlorophthalide,
20. 3,3-Bis[2-(p-pyrrolidinophenyl)-2-(p-methoxyphenyl)ethenyl]-5,6-dichloro-4,7-dibromophthalide,
2l. 3,3-Bis[2-(p-pyrrolidinophenyl)-2-(p-methylphenyl)ethenyl]-5-chloro-4,6,7-tribromophthalide,
22. 3,3-Bis[2-(p-pyrrolidinophenyl)-2-(p-methylphenyl)ethenyl]-4,5,6,7-tetrabromophthalide,
23. 3,3-Bis[2-(p-pyrrolidinophenyl)-2-(o-methyl-p-methoxyphenyl)ethenyl]-4,5,6,7-tetrachlorophthalide,
24. 3,3-Bis[2-(p-pyrrolidinophenyl)-2-(3,4-dimethylphenyl)ethenyl]-4,5,6,7-tetrabromophthalide,
25. 3,3-Bis[2-(p-pyrrolidino-o-methylphenyl)-2-(p-methylphenyl)ethenyl]-4,5,6,7-tetrachlorophthalide,
26. 3,3-Bis[2-(p-pyrrolidinophenyl)-2-(p-ethylphenyl)ethenyl]-4,5,6,7-tetrachlorophthalide,
27. 3,3-Bis[2-(p-pyrrolidino-o-chlorophenyl)-2-(p-methoxyphenyl)ethenyl]-4,5,6,7-tetrachlorophthalide,
28. 3,3-Bis[2-(p-2,5-dimethylpyrrolidinophenyl)-2-(p-methylphenyl)ethenyl]-4,5,6,7-tetrachlorophthalide,
29. 3,3-Bis[2-(p-piperidinophenyl)-2-(p-methoxyphenyl)ethenyl]-4,5,6,7-tetrachlorophthalide,
30. 3,3-Bis[2-(p-piperidinophenyl)-2-(p-methylphenyl)ethenyl]-4,5,6,7-tetrachlorophthalide,
31 3,3-Bis[2-(p-piperidinophenyl)-2-(p-methylphenyl)ethenyl]-4,5,6,7-tetrabromophthalide,
32. 3,3-Bis[2-(p-2-methylpiperidinophenyl)-2-phenylethenyl]-4,5,6,7-tetrachlorophthalide,
33. 3,3-Bis[2-(p-4-methylpiperidinophenyl)-2-(p-methoxyphenyl)ethenyl]-4,5,6,7-tetrachlorophthalide,
34. 3,3-Bis[2-(p-hexamethyleneiminophenyl)-2-(p-methylphenyl)ethenyl]-4,5,6,7-tetrachlorophthalide.
35. 3,3-Bis[2-(p-morpholinophenyl)-2-(p-methoxyphenyl)ethenyl]-4,5,6,7-tetrachlorophthalide,
36. 3,3-Bis[2-(p-pyrrodinophenyl)-2-(p-octylphenyl)ethenyl]4,5,6,7-tetrachlorophthalide,
37. 3,3-Bis[2-(l-ethylindolin-5-yl)-2-(p-methylphenyl)ethenyl]-4,5,6,7-tetrachlorophthalide,

38. 3,3-Bis[2-(l-methylindolin-5-yl)-2-(p-methoxyphenyl)ethenyl]-4,5,6,7-tetrachlorophthalide,
39. 3,3-Bis[2-(l,2,3,4-tetrahydroquinolin-6-yl)-2-(p-methylphenyl)ethenyl]-4,5,6,7-tetrachlorophthalide,
40. 3,3-Bis[2-(l-methoxyethyl-l,2,3,4-tetrahydroquinolin-6-yl)-2-(p-methylphenyl)ethenyl]-4,5,6,7-tetrabromophthalide,
41 3,3-Bis[2-durrolidinyl-2-(p-ethoxyphenyl)ethenyl]-4,5,6,7-tetrachlorophthalide,
42. 3,3-Bis[2-(p-pyrrolidinophenyl)-2-(p-methoxyethoxyphenyl)ethenyl]-4,5,6,7-tetrachlorophthalide,
43. 3,3-Bis[2-(p-pyrrolidinophenyl)-2-(p-allyloxyphenyl)ethenyl]-4,5,6,7-tetrachlorophthalide,
44. 3,3-Bis[2-(p-pyrrolidinophenyl)-2-(p-chloropropoxyphenyl)ethenyl]-4,5,6,7-tetrachlorophthalide,
45. 3,3-Bis[2-(p-pyrrolidinophenyl)-2-(p-cyclopentyloxyphenyl)ethenyl]-4,5,6,7-tetrachlorophthalide,
46. 3,3-Bis[2-(p-pyrrolidinophenyl)-2-(p-cyclohexyloxyphenyl)ethenyl]-4,5,6,7-tetrachlorophthalide,
47. 3,3-Bis[2-(p-pyrrolidinophenyl)-2-(p-benzyloxyphenyl)ethenyl]-4,5,6,7-tetrachlorophthalide,
48. 3,3-Bis[2-(p-pyrrolidinophenyl)-2-(p-phenoxyphenyl)ethenyl]-4,5,6,7-tetrachlorophthalide, and
49. 3,3-Bis{2-(p-pyrrolidinophenyl)-2-[p-(p-methoxyphenoxy)phenyl]ethenyl}-4,5,6,7-tetrachlorophthalide.

The divinyl compounds according to the present invention can be synthesized by the method shown below.

As a first step, an ethylene derivative represented by the formula (2) is synthesized from a ketone by one of the following Grignard reactions a, b and c:

a. 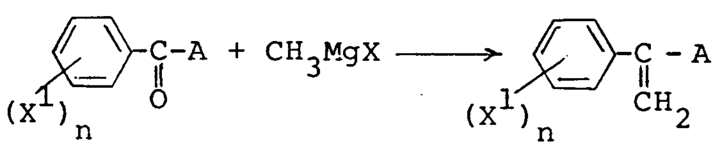 (2)

b. 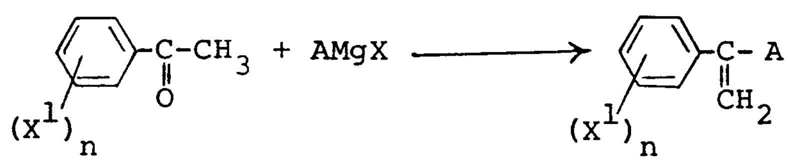 (2) and

c. 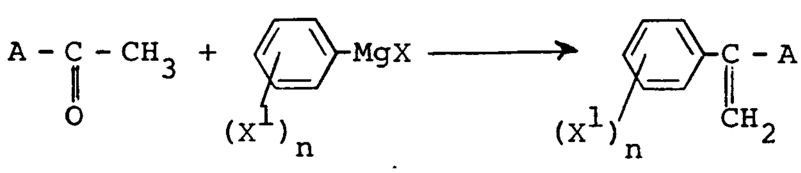 (2),

wherein A, $X^1$ and n are as defined above and X represents a halogen atom.

The ethylene derivative of formula (2) and a phthalic acid derivative of formula (3) (see below) are condensed in a molar ratio of 2:1 and in the presence of a dehydrating agent such as acetic anhydride or sulfuric acid. The reaction product is then purified to obtain the divinyl compounds represented by the formula (I) are obtained as nearly colorless crystals.

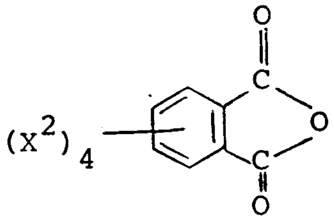 (3)

wherein $X^2$ is as defined above.

Thus the present invention also provides a process for the preparation of a divinyl compound represented by the formula (I), which process comprises condensing an ethylene derivative of formula (2) with a phthalic acid derivative of formula (3) in a molar ratio of 2:1 and in the presence of a dehydrating agent.

Examples of the ethylene derivative represented by the formula (2) are:

1. 1-phenyl-1-(p-pyrrolidinophenyl)ethylene,
2. 1-(p-methylphenyl)-1-(p-pyrrolidinophenyl)ethylene,
3. 1-(p-methoxyphenyl)-1-(p-pyrrolidinophenyl)ethylene,
4. 1-(p-ethoxyphenyl)-1-(p-pyrrolidinophenyl)ethylene,
5. 1-(p-butoxyphenyl)-1-(p-pyrrolidinophenyl)ethylene,
6. 1-(p-methoxyphenyl)-1-(p-pyrrolidino-o-methylphenyl)ethylene,
7. 1-(p-methoxyphenyl)-1-(p-pyrrolidino-o-methoxyphenyl)ethylene,
8. 1-(2,4-dimethylphenyl)-1-(p-pyrrolidinophenyl)ethylene,
9. 1-(2,4-dimethoxyphenyl)-1-(p-pyrrolidinophenyl)ethylene,
10. 1-(p-chlorophenyl)-1-(p-pyrrolidinophenyl)ethylene,
11. 1-(p-methylphenyl)-1-(p-2,5-dimethylpyrrolidinophenyl)ethylene,
12. 1-(p-methylphenyl)-1-(p-piperidinophenyl)ethylene,
13. 1-(p-methoxyphenyl)-1-(p-4-methylpiperidinophenyl)ethylene,
14. 1-(p-methylphenyl)-1-(p-hexamethyleneiminophenyl)ethylene,
15. 1-(p-methoxyphenyl)-1-(p-morpholinophenyl)ethylene,
16. 1-(p-methoxyphenyl)-1-(1-methylindoline-5-yl)ethylene,
17. 1-(p-methoxyphenyl)-1-(1-methyl-1,2,3,4-tetrahydroquinolin-6-yl)ethylene, and
18. 1-(p-methoxyphenyl)-1-durrolidinylethylene.

Examples of the phthalic acid derivatives represented by the formula (3) are:

tetrachlorophthalic anhydride; 4-chloro-3,5,6-tribromophthalic anhydride; 4,5-dichloro-3,6-dibromophthalic anhydride; 4-bromo-3,5,6-trichlorophthalic anhydride; 4,5-dibromo-3,6-dichlorophthalic anhydride; tetrabromophthalic anhydride; tetrafluorophthalic anhydride; tetraiodophthalic anhydride; 4,5-dichloro-3,6-difluorophthalic anhydride; 4-chloro-3,5,6-triiodophthalic anhydride and 4,5-dichloro-3,6-diiodophthalic anhydride.

The present invention also provides a recording material incorporating, as a chromogenic agent, a divinyl compound represented by the formula (I) as defined above.

In case where a pressure-sensitive recording paper or a heat-sensitive recording paper, for example, is produced with these divinyl compounds, one or more of the compounds can be used. By mixing not less than two of the compounds, the chromogenic property and the image stability are improved. Moreover, to improve the hue and the concentration of developed color, and the stability of the color image, various known chromogenic agents which give various hues can be used with the present compounds to the extent that they do not damage the facilities of the present compounds.

For instance, the present compound can be used with a chromogenic agent which has a fundamental skeleton of 3,3-bis(aminophenyl)-6-aminophthalide, 3,3-bis(indolyl)phthalide, 3-aminofluoran, aminobenzofluoran, 2,6-diaminofluoran, 2,6-diamino-3-methylfluoran, spiropyrane, phenothiazine, phenoxazine, leucoauramine, diarylcarbazolylmethane, 3-indolyl-3-(aminophenyl)azaphthalide, triaminofluorenephthalide or tetraaminodivinylphthalide.

When producing a pressure-sensitive recording paper, various solvents for the chromogenic agent can be used, such as those of the alkylbenzene series, alkylbiphenyl series, alkylnaphthalene series, diarylethane series, hydrogenated terphenyl series and chlorinated paraffin series. These can be used singly or as a mixture. For encapsulation, a coacervation method, an interfacial polymerization method or an in-situ method can be used. The divinyl compound may be encapsulated in solution within microcapsules present on a face of a paper sheet and an opposing face of another paper sheet is coated with a developer. Alternatively a mixture comprising the divinyl compound, a binder and a developer may coat the faces of a paper sheet.

As a developer, clays such as bentonite, activated clay or acid clay; metal salts of salicyclic acid, salicylic ester derivatives or salicylic acid derivatives,; hydroxy compounds such as 2,2-bis(p-hydroxyphenyl)-propane (bisphenol A) or esters of p-hydroxybenzoic acid; p-phenylphenol-formaldehyde resin, p-octylphenolformaldehyde resin and metal salts thereof are for example used.

As a developer, one or more of the following hydroxy compounds can, for example, be used:

p-phenylphenol, p-hydroxydiphenyl ether, methyl p-hydroxybenzoate, benzyl p-hydroxybenzoate, 2,2-bis(p-hydroxyphenyl)-propane, 4,4'-thiodiphenol, bis-(4-hydroxy-3-methylphenyl) sulfide, 4,4'-dihydroxydiphenylsulfone, 4-hydroxy-4'-methyldiphenylsulfone, 4-hydroxy-4'-ethyldiphenylsulfone, 3,4-dihydroxy-4'-methyldiphenylsulfone, 4-hydroxy-4'-isopropoxydiphenylsulfone, 4,4'-dihydroxy-3,3'-dimethyldiphenylsulfone, 4,4'-dihydroxy-3,3'-diallyldiphenylsulfone, 1,5-di(4-hydroxyphenylthio)-3-oxapentane, 1,7-di(4-hydroxyphenylthio)-3,5-dioxaheptane, 1,8-di(4-hydroxyphenylthio)-3,6-dioxaoctane and bis(4-hydroxy-3-methylphenyl) sulfide.

When producing a heat-sensitive recording paper, polyvinyl alcohol, methylcellulose, hydroxy-ethylcellulose, carboxymethylcellulose, gum arabic, gelatine, caseine, starch, polyvinyl pyrrolidone, copolymer of styrene and maleic anhydride, can, for example, be used as a binder.

Examples of sensitivity-improving agents are acetoanilide; paraffin wax; carnauba wax; higher fatty acids; esters of a higher fatty acid; amides of a higher fatty acid; phthalic esters; terephthalic esters; benzyl 4-benzyloxybenzoate; naphthol benzyl ether; 1,4-dialkoxynaphthalene; m-terphenyl; p-benzylbiphenyl, dibenzylbenzene; esters of 1-hydroxy-2-naphthoic acid; 1-phenoxy-2-naphthoxy-1-ethane; 1,2-di(3-methylphenoxy)ethane; 1-(2-isopropylphenoxy)-2-naphthoxy-2-ethane; esters of 2-hydroxy-3-naphthoic acid; 4,4'-dialkoxydiphenylsulfone; benzamide; diphenylamine, benzenesulfonamide; benzenesulfonanilide; carbazole, hydroquinone dibenzyl ether; and diphenyl carbonate. These can be used singly or after mixing together.

In order to improve the light-resistance and the preservability of the color image, it is effective to add an anti-oxidant, an anti-deteriorant or an ultraviolet absorbent, or to use a coating of a high polymeric substance.

The present invention also provides the use as a chromogenic agent of a divinyl compound represented by the formula (I) as defined above. The present invention is further described in the following Examples. The Synthetic Examples illustrated the preparation of compounds represented by the formula (I). The Production Examples illustrate the preparation of chromogenic recording-materials incorporating the compound represented by the formula (I).

The present invention also provides the use as a chromogenic agent of a divinyl compound represented by the formula (I) as defined above.

The present invention is further described in the following Examples. The Synthetic Examples illustrates the preparation of compounds represented by the formula (I). The Production Examples illustrate the preparation of chromogenic recording-materials incorporating the compound represented by the formula (I).

SYNTHETIC EXAMPLE 1:

Synthesis of 3,3-bis[2-(p-pyrrolidinophenyl)-2-(p-methoxyphenyl)ethenyl]-4,5,6,7-tetrachlorophthalide.

Into a mixture of 25 ml of acetic anhydride and 75 ml of o-dichlorobenzene, 14.0 g of 1-(p-methoxyphenyl)-1-(p-pyrrolidinophenyl)ethylene (m.p. 115-118 °C) and 21.5 g of tetrachlorophthalic anhydride were added and the mixture was stirred for 6 hours at 120 °C. Into 200 ml of water the reaction mixture was added and after making the mixture alkaline by adding sodium hydroxide, the alkaline reaction mixture was extracted with 70 ml of toluene. The solid matter obtained by evaporating the toluene from the extract was recrystallized from acetone while purifying with activated carbon to obtain 17.6 g of pale yellow crystals melting at 159 to 161 °C. (Yield: 85.2%)

From elementary analysis and by the infrared absorption spectrum and the nuclear magnetic resonance spectrum of the product obtained, it was confirmed that the product was represented by the following formula:

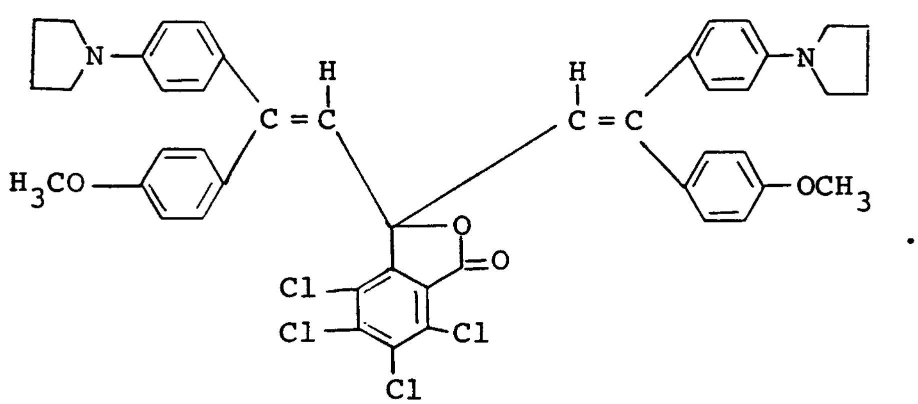

The compound was colored rapidly blue-black by activated clay. The $\lambda_{max}$ in methanol•stannic chloride was 900 nm.

The ethylene derivative, 1-(p-methoxyphenyl)-1-(p-pyrrolidinophenyl)ethylene, used in the above reaction, was synthesized as follows.

Into 30 ml of ether, 4 g of metallic magnesium were added and then 0.2 ml of methyl iodide was added to the mixture. After stirring the mixture for a while, a solution prepared by dissolving 24.8 g of methyl

iodide in 40 ml of ether was added to the mixture over 2 hours under a reflux condenser while stirring the mixture.

Separately, a solution was prepared by mixing 19.7 g of 4-methoxy-4'-pyrrolidinobenzophenone (melting at 152-154 °C) and 100 ml of tetrahydrofurane, and the solution was slowly added to the liquid reaction mixture. The mixture was stirred for one hour at a temperature of 40 to 50 °C and then mixed with 400 ml of water and 300 ml of toluene. After making the mixture weakly acidic by dilute hydrochloric acid, the acidified mixture was stirred for a while at 80 °C and separated into an aqueous layer and an organic layer (toluene layer). After adding activated carbon to the toluene layer and filtering the layer while hot, the toluene was distilled off from the filtrate to obtain 18.5 g of 1-(p-methylxyphenyl)-1-(p-pyrrolidinophenyl)-ethylene which was pale yellow in color.

SYNTHETIC EXAMPLE 2:
Synthesis of 3,3-bis[2-(p-pyrrolidinophenyl)-2-(p-ethoxyphenyl)ethenyl]-4,5,6,7-tetrachlorophthalide.

Into 50 ml of acetic anhydride 14.7 g of 1-(p-ethoxyphenyl)-1-(p-pyrrolidinophenyl)ethylene (m.p. 134 - 136 °C) and 14.3 g of tetrachlorophthalic anhydride were added and the mixture was stirred for 6 hours at 115 °C. Into 200 ml of water the reaction mixture was added and after making the mixture alkaline by adding sodium hydroxide, the alkaline reaction mixture was extracted with 70 ml of toluene. The solid matter obtained by evaporating the toluene from the extract was recrystallized from acetone while purifying with activated carbon to obtain 17.7 g of pale yellow crystals melting at 221 to 223 °C. (Yield: 82.9%)

From elementary analysis and the infrared absorption spectrum and the nuclear magnetic resonance spectrum of the product obtained, it was confirmed that the product was represented by the following formula:

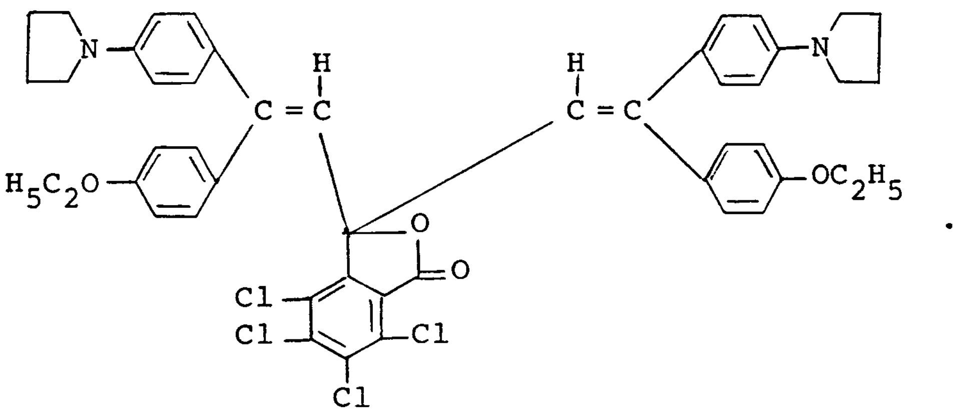

The compound was colored rapidly blue-black by activated clay. The $\lambda_{max}$ in methanol•stannic chloride was 897 nm.

SYNTHETIC EXAMPLE 3:
Synthesis of 3,3-bis{2-(p-pyrrolidinophenyl)-2-[p-(p-methoxyphenoxy)phenyl]ethenyl}-4,5,6,7-tetrachlorophthalide.

Into a mixture of 10 ml of acetic anhydride and 25 ml of o-dichlorobenzene, 14.3 g of tetrachlorophthalic anhydride was added and then 18.6 g of 1-[p-(p-methoxyphenoxy)phenyl]-1-(p-pyrrolidinophenyl)ethylene (m.p. 123.5 - 124.5 °C) was added dropwise for 1 hour at 120 °C and the mixture was stirred for 2 hours at the same temperature. Into 200 ml of water the reaction mixture was added and after making the mixture alkaline by adding sodium hydroxide, the alkaline reaction mixture was extracted with 70 ml of toluene. The solid matter obtained by evaporating the toluene from the extract was recrystallized from ethyl alcohol while purifying with activated carbon and obtained 18.2 g of pale yellow crystals melting at 95 °C (decomposed).

From elementary analysis, the infrared absorption spectrum and the nuclear magnetic resonance spectrum of the product obtained, it was confirmed that the product was represented by the following formula:

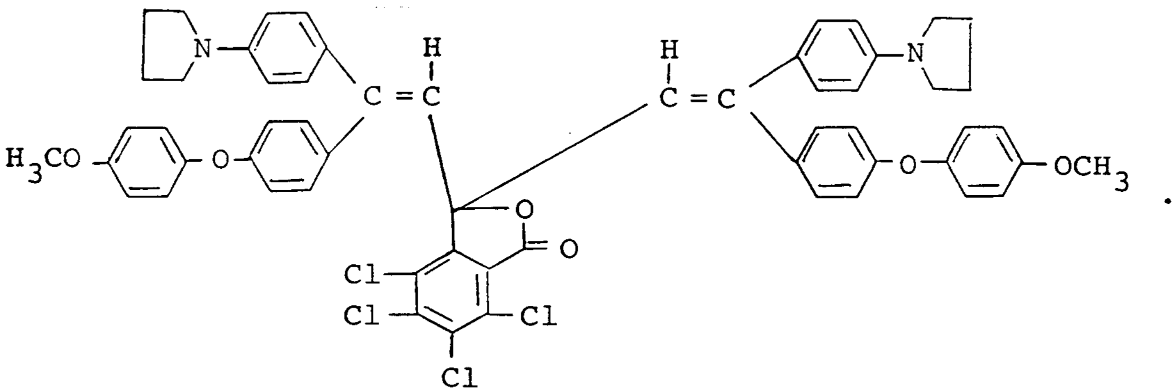

The compound was colored rapidly blue-black by activated clay. The $\lambda_{max}$ in methanol•stannic chloride was 910 nm.

SYNTHETIC EXAMPLES 4 to 43:

By reacting various ethylene derivatives with various phthalic acid derivatives in the same manner as in Synthetic Examples 1, 2 and 3, the divinyl compounds shown in Table 1 were synthesized. All the compounds were solid and colorless to pale yellow in color. They were colored rapidly into the hue shown in Table 1.

For preparing a pressure-sensitive recording paper with the divinyl compounds represented by the formula (I), any known method can be used, for instance, the coacervation method disclosed in US-A-2,800,458 US-A-2,806,457. For preparing the heat-sensitive recording paper, a known method, for instance, the method disclosed in JP-B-45-14039/1960, can be used.

TABLE 1 (I)

| Compound Number | A | $X^1$ | $X^2$ | Color | λmax (nm) | M. P. (°C) |
|---|---|---|---|---|---|---|
| 4 | 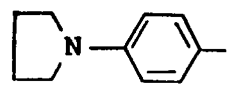 | p-n-$C_3H_7O$- | Cl | Blue black | 898 | 208 - 210 |
| 5 | Same as above | p-iso-$C_3H_7O$- | Cl | Blue black | 900 | 205 - 208 |
| 6 | Same as above | p-n-$C_4H_9O$- | Cl | Blue black | 900 | 168 - 171 |
| 7 | Same as above | p-i-$C_4H_9O$- | Cl | Blue black | 902 | 192 - 194 |
| 8 | Same as above | p-s-$C_4H_9O$- | Cl | Blue black | 900 | 157 - 160 |
| 9 | 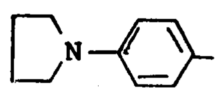 | p-t-$C_4H_9O$- | Cl | Blue black | 900 | 149 - 152 |
| 10 | Same as above | n-$CH_3O$-, m-$CH_3$- | Cl | Blue black | 898 | 165 - 167 |

TABLE 1 (II)

| Compound Number | A | $X^1$ | $X^2$ | Color | λmax (nm) | M. P. (°C) |
|---|---|---|---|---|---|---|
| 11 | Same as above | $3,4-(CH_3O)_2-$ | Cl | Blue black | 910 | 190 - 192 |
| 12 | Same as above | —— (n=0) | Cl | Black | 920 | 218 - 221 |
| 13 | Same as above | $p-CH_3-$ | Cl | Black | 910 | 152 ∿ 154 |
| 14 | Same as above | $p-CH_3O-$ | Br | Blue black | 904 | 162 ∿ 165 |
| 15 | Same as above | $n-C_5H_{11}O-$ | Cl | Blue black | 900 | 136 - 139 |
| 16 | 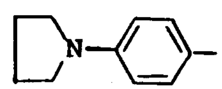 | $p-i-C_5H_{11}O-$ | Cl | Blue black | 902 | 118 ∿ 122 |
| 17 | Same as above | p-Cl- | Cl | Black | 930 | 95 ∿ 100 |

TABLE 1 (III)

| Compound Number | A | $X^1$ | $X^2$ | Color | λmax (nm) | M. P. (°C) |
|---|---|---|---|---|---|---|
| 18 | Same as above | p-$C_2H_5O$- | Br | Blue black | 902 | 217 - 219 |
| 19 | Same as above | p-$C_2H_5O$-, m-$CH_3O$- | Cl | Blue black | 910 | 145 ∿ 148 |
| 20 | Same as above | p-$C_2H_5O$-, m-$CH_3$- | Cl | Blue black | 900 | 170 - 173 |
| 21 | Same as above | p-$CH_3O$- | 5,6-$Cl_2$-, 4,7-$Br_2$- | Blue black | 902 | 164 - 166 |
| 22 | Same as above | p-$CH_3OC_2H_4O$- | Cl | Blue black | 905 | Difficult to crystallize |
| 23 | 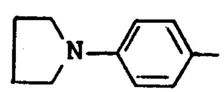 | p 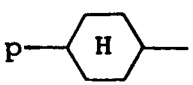 O — | Cl | Blue black | 902 | 135 - 138 |
| 24 | Same as above | p 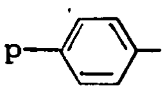 $CH_2O$- | Cl | Blue black | 903 | 151 - 155 |

TABLE 1 (IV)

| Compound Number | A | $X^1$ | $X^2$ | Color | λmax (nm) | M. P. (°C) |
|---|---|---|---|---|---|---|
| 25 | Same as above | p-$CH_3O$-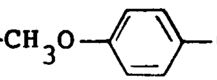-O- | Br | Blue black | 910 | Difficult to crystallize |
| 26 | Same as above | p-$CH_3O$- | 5-Cl-, $Br_3^-$ | Black | 900 | 135 - 138 |
| 27 | Same as above | 3,4-$(CH_3)_2$- | Cl | Black | 910 | 168 ∿ 170 |
| 28 | 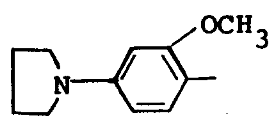 | p-$CH_3O$- | Cl | Black | 840 | Difficult to crystallize |
| 29 | 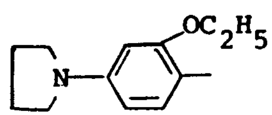 | p-$CH_3$- | Cl | Black | 850 | Difficult to crystallize |
| 30 | 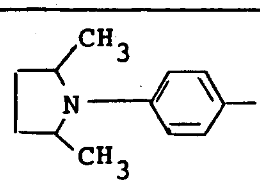 | p-$CH_3$- | Cl | Black | 910 | 128 - 131 |

TABLE 1 (V)

| Compound Number | A | $X^1$ | $X^2$ | Color | λmax (nm) | M. P. (°C) |
|---|---|---|---|---|---|---|
| 31 | 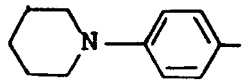 | $p-C_2H_5O-$ | Cl | Blue black | 890 | 180 ∿ 185 |
| 32 | 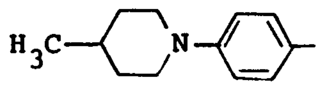 | $p-CH_3-$ | $5,6-Cl_2-$, $4,5-Br_2-$ | Black | 910 | Difficult to crystallize |
| 33 | 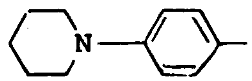 | $p-OCH_3-$ | Cl | Blue black | 905 | 180 - 184 |
| 34 | 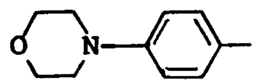 | ―― (n=0) | $Cl_4$ | Black | 910 | Difficult to crystallize |
| 35 | 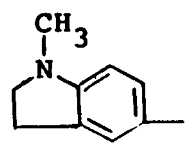 | $p-CH_3O-$ | Cl | Black | 910 | Difficult to crystallize |
| 36 | 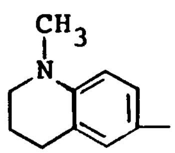 | $p-CH_3O-$ | Br | Black | 922 | 163 - 166 |

TABLE 1 (VI)

| Compound Number | A | $X^1$ | $X^2$ | Color | λmax (nm) | M. P. (°C) |
|---|---|---|---|---|---|---|
| 37 | 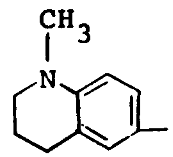 | p-$CH_3O$- | 5-Cl-, $Br_3$- | Black | 923 | Difficult to crystallize |
| 38 | 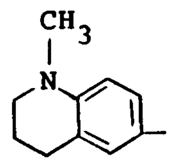 | p-$CH_3O$- | 5,6-$Cl_2$-, $Br_2$- | Black | 921 | 145 - 148 |
| 39 | Same as above | p-$CH_3O$- | Cl | Black | 915 | 150°C (decomposition) |
| 40 | 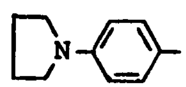 | p-n-$C_3H_7O$-, m-$CH_3$- | Cl | Blue black | 900 | 132 - 135 |
| 41 | Same as above | p-iso-$C_3H_7O$-, m-$CH_3$- | Cl | Blue black | 898 | 148 - 151 |
| 42 | Same as above | 3,4-$(C_2H_5O)_2$- | Cl | Blue black | 910 | 165 - 168 |
| 43 | Same as above | p-$C_8H_{17}O$- | Cl | Blue black | 900 | Difficult to crystallize |

PRODUCTION EXAMPLE 1:

Production of a pressure-sensitive copying paper.

Into 95 parts by weight of monoisopropylbiphenyl, 5 parts by weight of the compound of Example 2, namely, 3,3-bis[2-(p-pyrrolidinophenyl)-2-(p-ethoxyphenyl)ethenyl]-4,5,6,7-tetrachlorophthalide, were dissolved. A solution of 24 parts by weight of gelatine and 24 parts by weight of gum arabic in 400 parts by

weight of water, of which the pH was adjusted to 7, was added to the monoisopropylbiphenyl solution and the mixture was emulsified by a homogenizer. Into the emulsion, 100 parts by weight of warm water were added and after stirring the mixture for 30 minutes at 50° C about one part by weight of an aqueous 10% solution of sodium hydroxide was added. The mixture was further stirred for another 30 minutes at the same temperature.

In the next step, dilute acetic acid was added to the mixture to adjust the pH to 4.5 and after stirring for about one hour at 50° C the mixture was cooled to 0 to 5° C and stirred for 30 minutes. Then, 35 parts by weight of an aqueous 4% solution of glutaraldehyde were slowly added to the mixture to harden the resulting capsules and pH of the mixture was adjusted to 6 by adding a dilute aqueous solution of sodium hydroxide to complete the capsulation. During the operations, no coloring was observed.

The capsule suspension obtained was uniformly coated on a sheet of paper by a wire-bar in an amount such that the coated weight of capsules after drying was 6 $g/m^2$. The sheet was dried to obtain a capsule-coated paper sheet (the upper paper sheet).

On placing the upper paper sheet on a sheet of paper coated with a phenol-formaldehyde resin as a developer and applying writing pressure by a ball-pen on the sheets of paper, deep black letters rapidly appeared on the sheets.

The color image had excellent light-resistance and moisture-resistance and since the image had a strong absorption in the range of 800 to 1000 nm, it was possible to read the letters by OCR. Furthermore, the surface of the paper coated with the capsules had excellent light-resistance, and its color and chromogenic ability were not reduced by sunlight.

COMPARATIVE EXAMPLE 1:

In the same manner as in Production Example 1 except for using 5 parts by weight of the compound (D) as the chromogenic agent, a pressure-sensitive copying paper was prepared.

On subjecting the pressure-sensitive copying paper to color-development by a lower paper sheet on which a phenolformaldehyde resin had been applied, a light green image appeared slowly.

As the absorption of near infrared rays by the image was weak, it was difficult to read it by OCR (refer to Figure 1).

PRODUCTION EXAMPLE 2:

Production of a heat-sensitive recording paper:

1) Preparation of a liquid dispersion of a chromogenic agent (A-liquid):

A mixture of the following composition was pulverized by a paintshaker (made by TOYO-SEIKI Co., Ltd.) until the mean diameter of the particles of the chromogenic agent became 2 µm:
5 parts by weight of 3,3-bis[2-(p-pyrrodinophenyl)-2-(p-methoxyphenyl)ethenyl]-4,5,6,7-tetrachlorophthalide (Synthetic Example 1),
15 parts by weight of kaolin,
100 parts by weight of an aqueous 10% solution of polyvinyl alcohol and
85 parts by weight of water.

2) Preparation of a liquid dispersion of a developer and a sensitizer (B-liquid):

A mixture of the following composition was pulverized by a paintshaker until the mean diameter of the particles of developer and sensitizer became 3 µm:
15 parts by weight of bisphenol A,
10 parts by weight of zinc stearate,
15 parts by weight of stearic amide and
150 parts by weight of an aqueous 10% solution of polyvinyl alcohol.

3) Preparation and application of a liquid heat-sensitive material:

By mixing together 10 parts by weight of the A-liquid and 6.5 parts by weight of the B-liquid, a liquid heat-sensitive material was obtained. The liquid material was coated on a sheet of paper by a wire-bar uniformly in an amount such that the coated weight of solid materials after drying became 6 $g/m^2$. The

sheet of paper was dried to obtain a heat-sensitive recording paper.

The heat-sensitive recording paper was nearly colorless and did not show any spontaneous coloring (refer to Figure 3). The heat-sensitive recording paper showed a dark-black color after heating with a heated pen. The color image obtained had excellent light-resistance and moisture-resistance and as the color image had a strong absorption in the range of 700 to 1050 nm, it was possible to read the image by OCR.

The same results have been obtained using the compounds in other Synthetic Examples.

COMPARATIVE EXAMPLE 2:

In the same manner as in Production Example 2 except for using 5 parts by weight of the compound (A), a heat-sensitive recording paper was obtained. Although the heat-sensitive recording paper was colored black by heating with a heated pen, as the color image did not absorb any near infrared rays, it was impossible to read the color image by OCR (refer to Figure 2).

COMPARATIVE EXAMPLE 3:

In the same manner as in Production Example 2 except for using 5 parts by weight of the compound (B), a heat-sensitive recording paper was obtained. The heat-sensitive recording paper showed yellowish green spontaneous coloring. On heating the paper with a heated pen, a green color developed (refer to Figures 2 and 3).

From the above Production Examples and Comparative Examples, it is confirmed that the divinyl compound according to the present invention is an excellent chromogenic agent for recording materials.

## Claims

**1.** A divinyl compound represented by the formula (I):

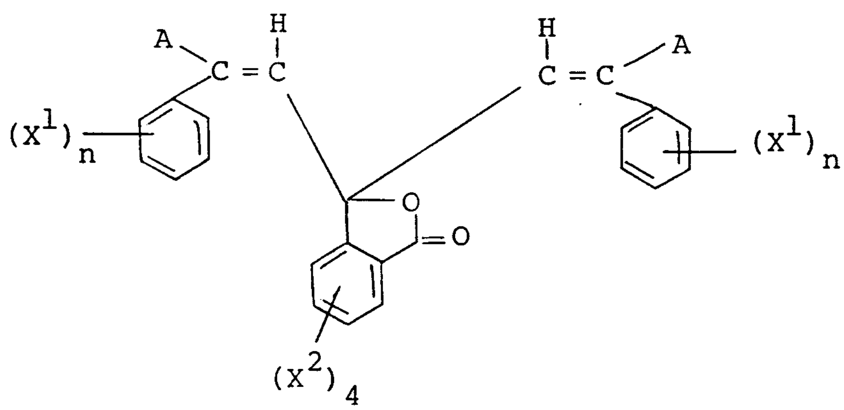

(I)

wherein A represents

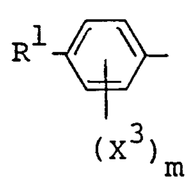 , 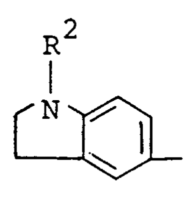 , 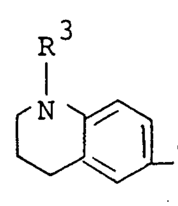 or

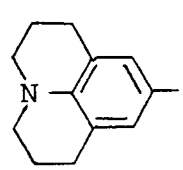 ;

$X^1$ represents an alkyl group, an alkoxy group, an alkoxyalkoxy group, an aryloxy group, a cycloalkoxy group, a haloalkoxy group, an alkenyloxy group, an aralkyloxy group or a halogen atom; $X^2$ represents a

halogen atom; $X^3$ represents an alkyl group having not more than 8 carbon atoms, an alkoxy group having not more than 8 carbon atoms or a halogen atom; $R^1$ represents a heterocyclic ring having one or more nitrogen atoms; $R^2$ and $R^3$ each represents a hydrogen atom, an alkyl group, an alkoxyalkyl group or a haloalkyl group; and m and n each represents 0, 1, 2 or 3; wherein each $X^1$ or each $X^3$ is the same or different when n or m respectively is 2 or 3 and each $X^2$ is the same or different.

**2.** A divinyl compound according to claim 1, wherein $R^1$ is

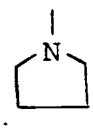, 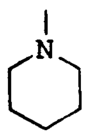, 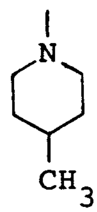, 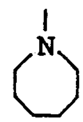, 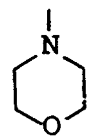 or

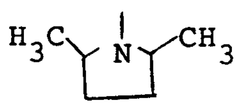 ;

$R^2$ is an alkyl group having not more than six carbon atoms; $R^3$ is an alkyl group having not more than six carbon atoms; $X^1$ is an alkyl group having not more than six carbon atoms, an alkoxy group having not more than six carbon atoms, a cyclohexyloxy group, a cyclopentyloxy group, a benzyloxy group, an allyloxy group, an alkoxyalkoxy group having not more than 6 carbon atoms in total, a phenoxy group, a phenoxy group substituted by an alkyl group or an alkoxy group or a halogen atom; $(X^2)_4$ represents four chlorine atoms, four bromine atoms, one chlorine atom and three bromine atoms or two chlorine atoms and two bromine atoms; $X^3$ is a methoxy group or an ethoxy group; m is 0 or 1 and n is 0, 1 or 2.

**3.** A divinyl compound according to claim 2, wherein $R^1$ is

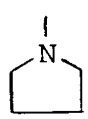

and m is 0.

**4.** A divinyl compound according to claim 3, wherein n is 1.

**5.** A divinyl compound according to claim 4, wherein $X^1$ is an alkoxy group having not more than four carbon atoms in a para-position.

**6.** A divinyl compound according to claim 1, wherein A is

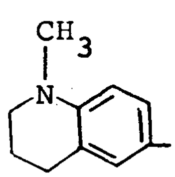,

$X^1$ is a p-methoxy group and n is 1.

**7.** 3,3-bis[2-(p-pyrrolidinophenyl)-2-(p-methoxyphenyl)ethenyl]-4,5,6,7-tetrachlorophthalide.

**8.** 3,3-bis[2-(p-pyrrolidinophenyl)-2-(p-ethoxyphenyl)ethenyl]-4,5,6,7-tetrachlorophthalide.

**9.** 3,3-bis[2-(p-pyrrolidinophenyl)-2-(p-propoxyphenyl)ethenyl]-4,5,6,7-tetrachlorophthalide.

**10.** 3,3-bis[2-(p-pyrrolidinophenyl)-2-(p-isopropoxyphenyl)ethenyl]-4,5,6,7-tetrachlorophthalide.

**11.** 3,3-bis[2-(p-pyrrolidinophenyl)-2-(p-butoxyphenyl)ethenyl]-4,5,6,7-tetrachlorophthalide.

**12.** 3,3-bis[2-(p-pyrrolidinophenyl)-2-(p-isobutoxyphenyl)ethenyl]-4,5,6,7-tetrachlorophthalide.

**13.** 3,3-bis[2-(p-pyrrolidinophenyl)-2-(p-sec-butoxyphenyl)ethenyl]-4,5,6,7-tetrachlorophthalide.

**14.** 3,3-bis[2-(p-pyrrolidinophenyl)-2-(p-tert-butoxyphenyl)ethenyl]-4,5,6,7-tetrachlorophthalide.

**15.** 3,3-bis[2-(p-pyrrolidinophenyl)-2-(m-methyl-p-methoxyphenyl)ethenyl]-4,5,6,7-tetrachlorophthalide.

**16.** 3,3-bis[2-(p-pyrrolidinophenyl)-2-(3,4-dimethoxyphenyl)ethenyl]-4,5,6,7-tetrachlorophthalide.

**17.** 3,3-bis[2-(p-pyrrolidinophenyl)-2-phenylethenyl]-4,5,6,7-tetrachlorophthalide.

**18.** 3,3-bis[2-(p-pyrrolidinophenyl)-2-(p-methylphenyl)ethenyl]-4,5,6,7-tetrachlorophthalide.

**19.** 3,3-bis[2-(p-pyrrolidinophenyl)-2-(p-methoxyphenyl)ethenyl]-4,5,6,7-tetrabromophthalide.

**20.** 3,3-bis[2-(p-pyrrolidinophenyl)-2-(pentyloxyphenyl)ethenyl]-4,5,6,7-tetrachlorophthalide.

**21.** 3,3-bis[2-(p-pyrrolidinophenyl)-2-(p-isopentyloxyphenyl)ethenyl]-4,5,6,7-tetrachlorophthalide.

**22.** 3,3-bis[2-(p-pyrrolidinophenyl)-2-(p-chlorophenyl)ethenyl]-4,5,6,7-tetrachlorophthalide.

**23.** 3,3-bis[2-(p-pyrrolidinophenyl)-2-(p-ethoxyphenyl)ethenyl]-4,5,6,7-tetrabromophthalide.

**24.** 3,3-bis[2-(p-pyrrolidinophenyl)-2-(m-methoxy-p-ethoxyphenyl)ethenyl]-4,5,6,7-tetrachlorophthalide.

**25.** 3,3-bis[2-(p-pyrrolidinophenyl)-2-(m-methyl-p-ethoxyphenyl)ethenyl]-4,5,6,7-tetrachlorophthalide.

**26.** 3,3-bis[2-(p-pyrrolidinophenyl)-2-(p-methoxyphenyl)ethenyl]-5,6-dichloro-4,7-dibromophthalide.

**27.** 3,3-bis{2-(p-pyrrolidinophenyl-2-[p-(p-methoxyphenoxy)phenyl]ethenyl}-4,5,6,7-tetrachlorophthalide.

**28.** 3,3-bis[2-(p-pyrrolidinophenyl)-2-(m-methyl-p-n-propoxyphenyl)ethenyl]-4,5,6,7-tetrachlorophthalide.

**29.** 3,3-bis[2-(p-pyrrolidinophenyl)-2-(m-methyl-p-iso-propoxyphenyl)ethenyl]-4,5,6,7-tetrachlorophthalide.

**30.** 3,3-bis[2-(p-pyrrolidinophenyl)-2-(3,4-diethoxyphenyl)ethenyl]-4,5,6,7-tetrachlorophthalide.

**31.** A process for the preparation of a divinyl compound represented by the formula (I) as defined in any one of the preceding claims, which process comprises condensing an ethylene derivative of formula (2):

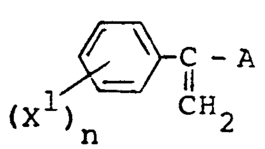

wherein A, $X^1$ and n are as defined in claim 1, with a phthalic acid derivative of formula (3):

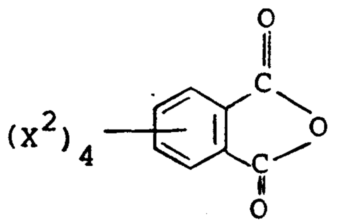

(3)

wherein $X^2$ is as defined in claim 1, in a molar ratio of 2:1 and in the presence of a dehydrating agent.

**32.** A process according to claim 31, wherein the ethylene derivative of formula (2) has been prepared by means of a Grignard reaction between the compounds $CH_3MgX$ and

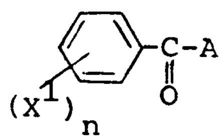

wherein X represents a halogen atom and A, $X^1$ and n are as defined in claim 31.

**33.** A process according to claim 31, wherein the ethylene derivative of formula (2) has been prepared by means of a Grignard reaction between the compounds AMgx and

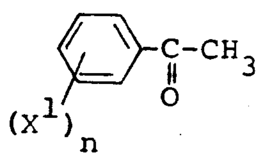

wherein X represents a halogen atom and A, $X^1$ and n are as defined in claim 31.

**34.** A process according to claim 31, wherein the ethylene derivative of formula (2) has been prepared by means of a Grignard reaction between the compounds

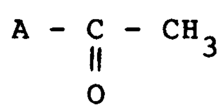

and

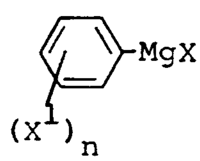

wherein X represents a halogen atom and A, $X^1$ and n are as defined in claim 31.

**35.** A recording material incorporating, as a chromogenic agent, a divinyl compound represented by the formula (I) as claimed in any one of claims 1 to 30.

**36.** A recording material according to claim 35, which is a pressure-sensitive recording paper wherein the divinyl compound is encapsulated in solution within microcapsules present on a face of a paper sheet and an opposing face of another paper sheet is coated with a developer.

**37.** A recording material according to claim 35, which is a heat sensitive recording paper wherein a mixture comprising the divinyl compound, a binder and a developer coats a face of a paper sheet.

**38.** Use as a chromogenic agent of a divinyl compound represented by the formula (I) as claimed in any one of claims 1 to 30.

**39.** A recording material according to any one of claims 35 to 37 incorporating at least two said divinyl compounds.

**40.** A recording material according to any one of claims 35 to 37 or 39, further comprising an additional chromogenic agent having the fundamental skeleton of 3,3-bis(aminophenyl)-6-aminophthalide, 3,3-bis-(indolyl)-phthalide, 3-aminofluoran, aminobenzofluoran, 2,6-diaminofluoran, 2,6-diamino-3-methylfluoran, spiropyrane, phenothiazine, phenoxazine, leucoauramine, diarylcarbazolylmethane, 3-indolyl-3-(aminophenyl)-azaphthalide, triaminofluorenephthalide or tetraaminodivinylphthalide.

## Revendications

**1.** Composé divinylique représenté par la formule I :

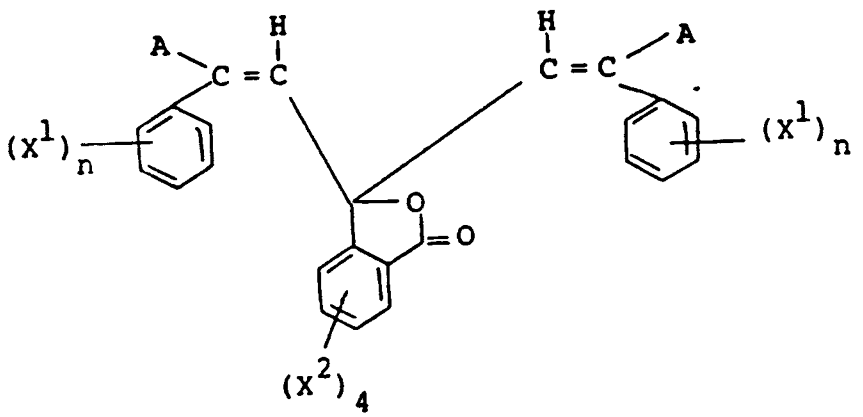

(I)

dans laquelle A représente :

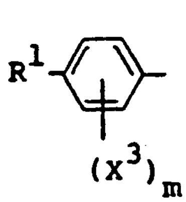 , 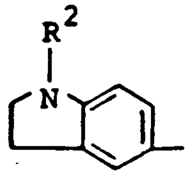 , 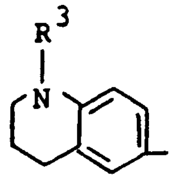 ou 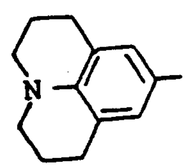 ;

dans laquelle $X^1$ représente un groupe alkyl, un groupe alkoxy, un groupe alkoxyalkoxy, un groupe aryloxy, un groupe cycloalkoxy, un groupe haloalkoxy, un groupe alkényloxy, un groupe aralkyloxy ou un atome d'halogène ; $X^2$ représente un atome d'halogène ; $X^3$ représente un groupe alkyl n'ayant pas plus de huit atomes de carbone, un groupe alkoxy n'ayant pas plus de huit atomes de carbone ou un atome d'halogène ; $R^1$ représente un cycle hétérocyclique ayant un ou plusieurs atomes d'azote ; $R^2$ et $R^3$ représentent chacun un atome d'hydrogène, un groupe alkyl, un groupe alkoxyalkyl ou un groupe haloalkyl et m et n représentent chacun 0, 1, 2 ou 3 ; dans lequel chacun des symboles $X^1$ ou chacun des symboles $X^3$ est identique ou différent lorsque n ou m respectivement est 2 ou 3 et chacun des

symboles $X^2$ est identique ou différent.

**2.** Composé divinylique selon la revendication 1, dans lequel $R^1$ est :

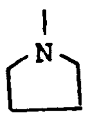, 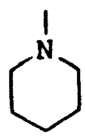, 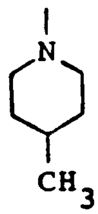, 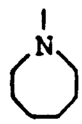, 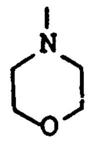 ou 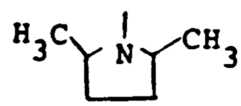

$R^2$ est un groupe alkyl n'ayant pas plus de six atomes de carbone ; $R^3$ est un groupe alkyl n'ayant pas plus de six atomes de carbone, $X^1$ est un groupe alkyl n'ayant pas plus de six atomes de carbone, un groupe alkoxy n'ayant pas plus de six atomes de carbone, un groupe cyclohexyloxy, un groupe cyclopentyloxy, un groupe benzyloxy, un groupe allyloxy, un groupe alkoxyalkoxy n'ayant pas plus de six atomes de carbone au total, un groupe phénoxy, un groupe phénoxy substitué par un groupe alkyl ou un groupe alkoxy ou un atome d'halogène ; $(X^2)_4$ représente quatre atomes de chlore, quatre atomes de brome, un atome de chlore et trois atomes de brome ou deux atomes de chlore et deux atomes de brome ; $X^3$ est un groupe méthoxy ou un groupe éthoxy ; m est 0 ou 1 et n est 0, 1 ou 2.

**3.** Composé divinylique selon la revendication 2, dans lequel $R^1$ est

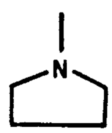

et m est 0.

**4.** Composé divinylique selon la revendication 3, dans lequel n est 1.

**5.** Composé divinylique selon la revendication 4, dans lequel $X^1$ est un groupe alkoxy n'ayant pas plus de quatre atomes de carbone en position para.

**6.** Composé divinylique selon la revendication 1, dans lequel A est

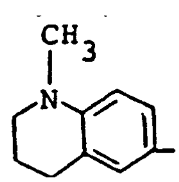,

$X^1$ est un groupe p-méthoxy et n est 1.

**7.** 3,3-bis[2-(p-pyrrolidinophényl)-2-(p-méthoxyphényl)éthényl]-4,5,7-tétrachlorophtalide.

**8.** 3,3-bis[2-(p-pyrrolidinophényl)-2-(p-éthoxyphényl)éthényl]-4,5,6,7-tétrachlorophtalide.

**9.** 3,3-bis[2-(p-pyrrolidinophényl)-2-(p-propoxyphényl)éthényl]-4,5,6,7-tétrachlorophtalide.

**10.** 3,3-bis[2-(p-pyrrolidinophényl)-2-(p-isopropoxyphényl)éthényl]-4,5,6,7-tétrachlorophtalide.

**11.** 3,3-bis[2-(p-pyrrolidinophényl)-2-(p-butoxyphényl)éthényl]-4,5,6,7-tétrachlorophtalide.

**12.** 3,3-bis[2-(p-pyrrolidinophényl)-2-(p-isobutoxyphényl)éthényl]-4,5,6,7-tétrachlorophtalide.

**13.** 3,3-bis[2-(p-pyrrolidinophényl)-2-(p-sec-butoxyphényl)éthényl]-4,5,6,7-tétrachlorophtalide.

**14.** 3,3-bis[2-(p-pyrrolidinophényl)-2-(p-tert-butoxyphényl)éthényl]-4,5,6,7-tétrachlorophtalide.

**15.** 3,3-bis[2-(p-pyrrolidinophényl)-2-(m-méthyl-p-méthoxyphényl)éthényl]-4,5,6,7-tétrachlorophtalide.

**16.** 3,3-bis[2-(p-pyrrolidinophényl)-2-(3,4-diméthoxyphényl)éthényl]-4,5,6,7-tétrachlorophtalide.

**17.** 3,3-bis[2-(p-pyrrolidinophényl)-2-phényléthényl]-4,5,6,7-tétrachlorophtalide.

**18.** 3,3-bis[2-(p-pyrrolidinophényl)-2-(p-méthylphényl)éthényl]-4,5,6,7-tétrachlorophtalide.

**19.** 3,3-bis[2-(p-pyrrolidinophényl)-2-(p-méthoxyphényl)éthényl]-4,5,6,7-tétrabromophtalide.

**20.** 3,3-bis[2-(p-pyrrolidinophényl)-2-(pentyloxyphényl)éthényl]-4,5,6,7-tétrachlorophtalide.

**21.** 3,3-bis[2-(p-pyrrolidinophényl)-2-(p-isopentyloxyphényl)éthényl]-4,5,6,7-tétrachlorophtalide.

**22.** 3,3-bis[2-(p-pyrrolidinophényl)-2-(p-chlorophényl)éthényl]-4,5,6,7-tétrachlorophtalide.

**23.** 3,3-bis[2-(p-pyrrolidinophényl)-2-(p-éthoxyphényl)éthényl]-4,5,6,7-tétrabromophtalide.

**24.** 3,3-bis[2-(p-pyrrolidinophényl)-2-(m-méthoxy-p-éthoxyphényl)éthényl]4,5,6,7-tétrachlorophtalide.

**25.** 3,3-bis[2-(p-pyrrolidinophényl)-2-(m-méthyl-p-éthoxyphényl)éthényl]-4,5,6,7-tétrachlorophtalide.

**26.** 3,3-bis[2-(p-pyrrolidinophényl)-2-(p-méthoxyphényl)éthényl]-5,6-dichloro-4,7-dibromophtahide.

**27.** 3,3-bis{2-(p-pyrrolidinophényl-2-[p-(p-méthoxyphénoxy)phényl]éthényl}-4,5,6,7-tétrachlorophtalide.

**28.** 3,3-bis[2-(p-pyrrolidinophényl)-2-(m-méthyl-p-n-propoxyphényl)éthényl]-4,5,6,7-tétrachlorophtalide.

**29.** 3,3-bis[2-(p-pyrrolidinophényl)-2-(m-méthyl-p-iso-propoxyphényl)éthényl]-4,5,6,7-tétrachlorophtalide.

**30.** 3,3-bis[2-(p-pyrrolidinophényl)-2-(3,4-diéthoxyphényl)éthényl]-4,5,6,7-tétrachlorophtalide.

**31.** Procédé pour la préparation d'un composé divinylique représenté par la formule I, telle que définie dans l'une quelconque des revendications précédentes, lequel procédé comprend la condensation d'un dérivé de l'éthylène répondant à la formule II :

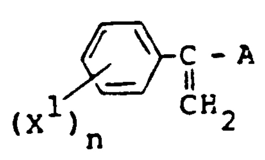

dans laquelle A, $X^1$ et n sont tels que définis dans la revendication 1, avec un dérivé de l'acide phtalique répondant à la formule III :

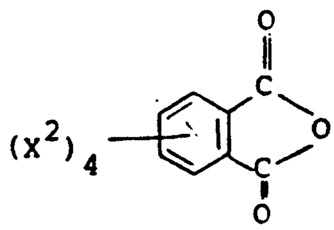

dans laquelle $X^2$ est tel que défini dans la revendication 1, dans un rapport molaire de 2:1 et en présence d'un agent déshydratant.

**32.** Procédé selon la revendication 31, dans lequel le dérivé de l'éthylène répondant à la formule II a été préparé par une réaction de GRIGNARD entre les composés $CH_3MgX$ et

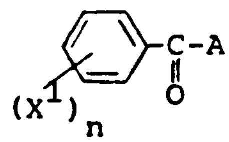

dans laquelle X représente un atome d'halogène et A, $X^1$ et n sont tels que définis dans la revendication 31.

**33.** Procédé selon la revendication 31, dans lequel le dérivé de l'éthylène répondant à la formule II a été préparé par une réaction de GRIGNARD entre les composés AMgX et

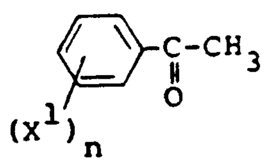

dans laquelle X représente un atome d'halogène et A, $X^1$ et n sont tels que définis dans la revendication 31.

**34.** Procédé selon la revendication 31, dans lequel le dérivé de l'éthylène répondant à la formule II a été préparé par une réaction de GRIGNARD entre les composés

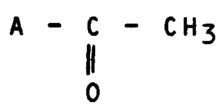

et

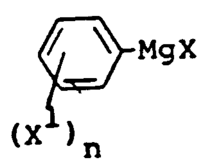

dans laquelle X représente un atome d'halogène et A, $X^1$ et n sont tels que définis dans la revendication 31.

**35.** Matière d'enregistrement contenant comme agent chromogène un composé divinylique représenté par la formule I tel que revendiqué dans l'une quelconque des revendications 1 à 30.

**36.** Matière d'enregistrement selon la revendication 35 qui est un papier d'enregistrement sensible à la pression dans lequel le composé divinylique est encapsulé en solution dans des microcapsules présentent sur une face de la feuille de papier et la face opposée d'une autre feuille de papier est revêtu d'un révélateur.

**37.** Matière d'enregistrement selon la revendication 35 qui est un papier d'enregistrement thermosensible dans lequel un mélange comprenant le composé divinylique, un liant et un révélateur revêt une face

d'une feuille de papier.

**38.** Utilisation comme agent chromogène d'un composé divinylique représenté par la formule I selon l'une quelconque des revendications 1 à 30.

**39.** Matière d'enregistrement selon l'une quelconque des revendications 35 à 37, contenant au moins deux de ces composés divinylique.

**40.** Matière d'enregistrement selon l'une quelconque des revendications 35 à 37 ou 39, comprenant en outre un agent chromogène supplémentaire ayant le squelette fondamental du 3,3-bis(aminophényl)-6-aminophtalide, du 3,3-bis(indolyl)-phtalide, du 3-aminofluorane, de l'aminobenzofluorane, du 2,6-diaminofluorane, du 2,6-diamino-3-méthylfluorane, du spiropyrane, de la phénothiazine, de la phénoxazine, de la leucoauramine, du diarylcarbazolylméthane, du 3-indolyl-3-(aminophényl)-azaphtalide, du triaminofluorènephtalide ou du tétraaminodivinylphtalide.

**Patentansprüche**

**1.** Divinylverbindung, dargestellt durch die Formel (I)

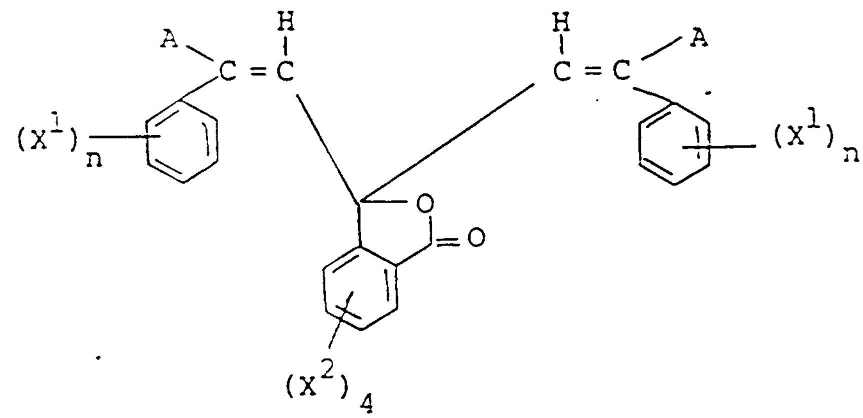

(I)

worin bedeuten: A

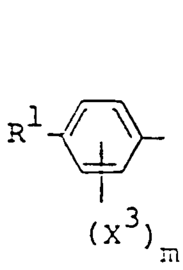
,
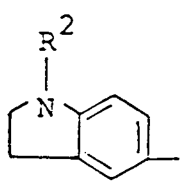
,
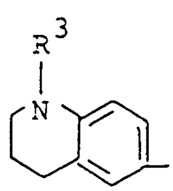
oder
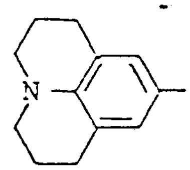

$X^1$ eine Alkylgruppe, eine Alkoxygruppe, eine Alkoxyalkoxygruppe, eine Aryloxygruppe, eine Cycloalkoxygruppe, eine Haloalkoxygruppe, eine Alkenyloxygruppe, eine Aralkyloxygruppe oder ein Halogenatom; $X^2$ ein Halogenatom; $X^3$ eine Alkylgruppe mit nicht mehr als 8 Kohlenstoffatomen, eine Alkoxygruppe mit nicht mehr als 8 Kohlenstoffatomen oder ein Halogenatom; $R^1$ einen heterozyklischen Ring mit einem oder mehreren Stickstoffatomen; $R^2$ und $R^3$ jeweils ein Wasserstoffatom, eine Alkylgruppe, eine Alkoxyalkylgruppe oder eine Haloalkylgruppe; und m und n jeweils 0, 1, 2 oder 3, worin jedes $X^1$ oder jedes $X^3$ gleich oder verschieden sind, wenn n bzw. m 2 oder 3 bedeutet und wobei jedes $X^2$ gleich oder verschieden ist.

**2.** Divinylverbindung nach Anspruch 1, worin bedeuten: $R^1$

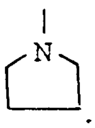, 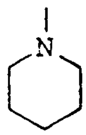, 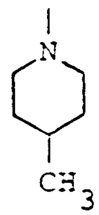, 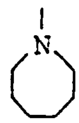, 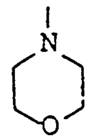 oder 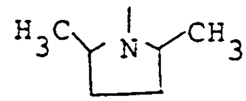

$R^2$ eine Alkylgruppe mit nicht mehr als 6 Kohlenstoffatomen; $R^3$ eine Alkylgruppe mit nicht mehr als 6 Kohlenstoffatomen; $X^1$ eine Alkylgruppe mit nicht mehr als 6 Kohlenstoffatomen, eine Alkoxygruppe mit nicht mehr als 6 Kohlenstoffatomen, eine Cyclohexyloxygruppe, eine Cyclopentyloxygruppe, eine Benzyloxygruppe, eine Allyloxygruppe, eine Alkoxyalkoxygruppe mit insgesamt nicht mehr als 6 Kohlenstoffatomen, eine Phenoxygruppe, eine Phenoxygruppe, substituiert durch eine Alkylgruppe oder eine Alkoxygruppe oder ein Halogenatom; $(X^2)_4$ 4 Chloratome, 4 Bromatome, 1 Chloratom und 3 Bromatome oder 2 Chloratome und 2 Bromatome; $X^3$ eine Methoxygruppe oder eine Ethoxygruppe; m 0 oder 1 und n 0, 1 oder 2.

**3.** Divinylverbindung nach Anspruch 2, dadurch **gekennzeichnet,** dass $R^1$

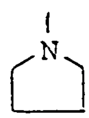

und m 0 sind.

**4.** Divinylverbindung nach Anspruch 3, dadurch **gekennzeichnet,** dass n 1 ist.

**5.** Divinylverbindung nach Anspruch 4, dadurch **gekennzeichnet,** dass $X^1$ eine Alkoxygruppe mit nicht mehr als 4 Kohlenstoffatomen in der para-Position ist.

**6.** Divinylverbindung nach Anspruch 1, dadurch **gekennzeichnet**, dass A

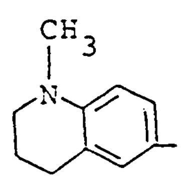

$X^1$ eine p-Methoxygruppe und n 1 sind.

**7.** 3,3-Bis(2-(p-pyrrolidinophenyl)-2-(p-methoxyphenyl)ethenyl)-4,5,6,7-tetrachlorophthalid.

**8.** 3,3-Bis(2-(p-pyrrolidinophenyl)-2-(p-ethoxyphenyl)ethenyl)-4,5,6,7-tetrachlorphthalid.

**9.** 3,3-Bis(2-(p-pyrrolidinophenyl)-2-(p-propoxyphenyl)ethenyl)-4,5,6,7-tetrachlorophthalid.

**10.** 3,3-Bis(2-(p-pyrrolidinophenyl)-2-(p-isopropoxyphenyl)-ethenyl)-4,5,6,7-tetrachlorophthalid.

**11.** 3,3-Bis(2-(p-pyrrolidinophenyl)-2-(p-butoxyphenyl)ethenyl)-4,5,6,7-tetrachlorophthalid.

**12.** 3,3-Bis(2-(p-pyrrolidinophenyl)-2-(p-isobutoxyphenyl)ethenyl)-4,5,6,7-tetrachlorophthalid.

**13.** 3,3-Bis(2-(p-pyrrolidinophenyl)-2-(p-sek.-butoxyphenyl)ethenyl)-4,5,6,7-tetrachlorophthalid.

**14.** 3,3-Bis(2-(p-pyrrolidinophenyl)-2-(p-tert.-butoxyphenyl)ethenyl)-4,5,6,7-tetrachlorophthalid.

**15.** 3,3-Bis(2-(p-pyrrolidinophenyl)-2-(m-methyl-p-methoxyphenyl)ethenyl)-4,5,6,7-tetrachlorophthalid.

**16.** 3,3-Bis(2-(p-pyrrolidinophenyl)-2-(3,4-dimethoxyphenyl)ethenyl)-4,5,6,7-tetrachlorophthalid.

**17.** 3,3-Bis(2-(p-pyrrolidinophenyl)-2-phenylethenyl)-4,5,6,7-tetrachlorophthalid.

**18.** 3,3-Bis(2-(p-pyrrolidinophenyl)-2-(p-methylphenyl)ethenyl)-4,5,6,7-tetrachlorophthalid.

**19.** 3,3-Bis(2-(p-pyrrolidinophenyl)-2-(p-methoxyphenyl)ethenyl)-4,5,6,7-tetrabromophthalid.

**20.** 3,3-Bis(2-(p-pyrrolidinophenyl)-2-(pentyloxyphenyl)ethenyl)-4,5,6,7-tetrachlorophthalid.

**21.** 3,3-Bis(2-(p-pyrrolidinophenyl)-2-(p-isopentyloxyphenyl)ethenyl)-4,5,6,7-tetrachlorophthalid.

**22.** 3,3-Bis(2-(p-pyrrolidinophenyl)-2-(p-chlorophenyl)ethenyl)-4,5,6,7-tetrachlorophthalid.

**23.** 3,3-Bis(2-(p-pyrrolidinophenyl)-2-(p-ethoxyphenyl)ethenyl)-4,5,6,7-tetrabromophthalid.

**24.** 3,3-Bis(2-(p-pyrrolidinophenyl)-2-(m-methoxy-p-ethoxyphenyl)ethenyl)-4,5,6,7-tetrachlorophthalid.

**25.** 3,3-Bis(2-(p-pyrrolidinophenyl)-2-(m-methyl-p-ethoxyphenyl)ethenyl)-4,5,6,7-tetrachlorophthalid.

**26.** 3,3-Bis(2-(p-pyrrolidinophenyl)-2-(p-methoxyphenyl)ethenyl)-5,6-dichloro-4,7-dibromophthalid.

**27.** 3,3-Bis(2-(p-pyrrolidinophenyl-2-(p-methoxyphenoxy)phenyl)ethenyl)-4,5,6,7-tetrachlorophthalid.

**28.** 3,3-Bis(2-(p-pyrrolidinophenyl)-2-(m-methyl-p-n-propoxyphenyl)ethenyl)-4,5,6,7-tetrachlorophthalid.

**29.** 3,3-Bis(2-(p-pyrrolidinophenyl)-2-(m-methyl-p-isopropoxyphenyl)ethenyl)-4,5,6,7-tetrachlorophthalid.

**30.** 3,3-Bis(2-(p-pyrrolidinophenyl)-2-(3,4-diethoxyphenyl)ethenyl)-4,5,6,7-tetrachlorophthalid.

**31.** Verfahren zur Herstellung einer Divinylverbindung, dargestellt durch die Formel (I), nach einem der vorhergehenden Ansprüche, **gekennzeichnet** durch die Kondensation eines Ethylenderivats der Formel (II)

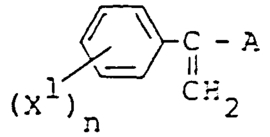

(II)

worin A, $X^1$ und n die in Anspruch 1 angegebenen Definitionen aufweisen, mit einem Phthalsäurederivat der Formel (III)

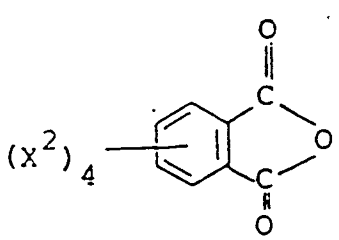

(III)

worin $X^2$ die in Anspruch 1 angegebene Bedeutung aufweist, in einem molaren Verhältnis von 2:1 und in Gegenwart eines Dehydratisierungsmittels.

**32.** Verfahren nach Anspruch 31, dadurch **gekennzeichnet,** dass das Ethylenderivat der Formel (II) mit Hilfe einer Grignard-Reaktion zwischen den Verbindungen $CH_3MgX$ und

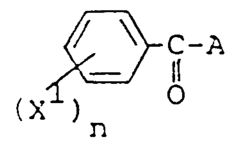

hergetellt ist, worin X ein Halogenatom bedeutet und worin A, $X^1$ und n die in Anspruch 31 angegebenen Definitionen aufweisen.

**33.** Verfahren nach Anspruch 31, dadurch **gekennzeichnet,** dass das Ethylenderivt der Formel (II) mit Hilfe einer Grignard-Reaktion zwischen den Verbindungen AMgX und

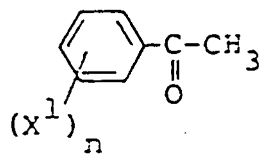

hergestellt worden ist, worin X ein Halogenatom bedeutet und worin A, $X^1$ und n die in Anspruch 31 angegebenen Definitionen aufweisen.

**34.** Verfahren nach Anspruch 31, dadurch **gekennzeichnet,** dass das Ethylenderivat der Formel (II) mit Hilfe einer Grignard-Reaktion zwischen den Verbindungen

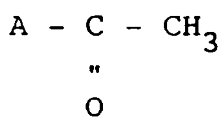

und

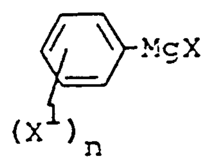

hergestellt worden ist, worin X ein Halogentom bedeutet und worin A, $X^1$ und n die in Anspruch 31 angegebenen Bedeutungen aufweisen.

**35.** Aufzeichnungsmaterial, umfassend als chromogenes Mittel eine Divinylverbindung, die durch die Formel (I) dargestellt ist, nach einem der Ansprüche 1 bis 30.

**36.** Aufzeichnungsmaterial nach Anspruch 35, dadurch **gekennzeichnet,** dass es ein druckempfindliches Aufzeichnungspapier ist, worin die Divinylverbindung in Lösung innerhalb von Mikrokapseln eingeschlossen ist, die auf einer Seite eines Papierblattes vorhanden sind und wobei eine gegenüberliegende Seite eines anderen Papierblattes mit einem Entwickler beschichtet ist.

**37.** Aufzeichnungsmaterial nach Anspruch 35, dadurch **gekennzeichnet,** dass es ein wärmeempfindliches Aufzeichnungspapier ist, wobei eine Mischung, die die Divinylverbindung, ein Bindemittel und einen Entwickler umfasst, auf einer Seite eines Papierblattes beschichtet ist.

**38.** Verwendung einer Divinylverbindung, die durch die Formel (I) dargestellt ist, nach einem der Ansprüche 1 bis 30 als ein chromogenes Mittel.

**39.** Aufzeichnungsmaterial nach einem der Ansprüche 35 bis 37, umfassend zumindest zwei der Divinylverbindungen.

**40.** Aufzeichnungsmaterial nach einem der Ansprüche 35 bis 37 oder 39, dadurch **gekennzeichnet,** dass

es weiterhin ein zusätzliches chromogenes Mittel umfasst, das das Grundgerüst von 3,3-Bis-(aminophenyl)-6-aminophthalid, 3,3-Bis(indolyl)-phthalid, 3-Aminofluoran, Aminobenzofluoran, 2,6-Diaminofluoran, 2,6-Diamino-3-methylfluoran, Spiropyran, Phenothiazin, Phenoxazin, Leukoauramin, Diarylcarbazolylmethan, 3-Indolyl-3-(aminophenyl)-azaphthalid, Triaminofluorenphthalid oder Tetraaminodivinylphthalid aufweist.

Figure 1

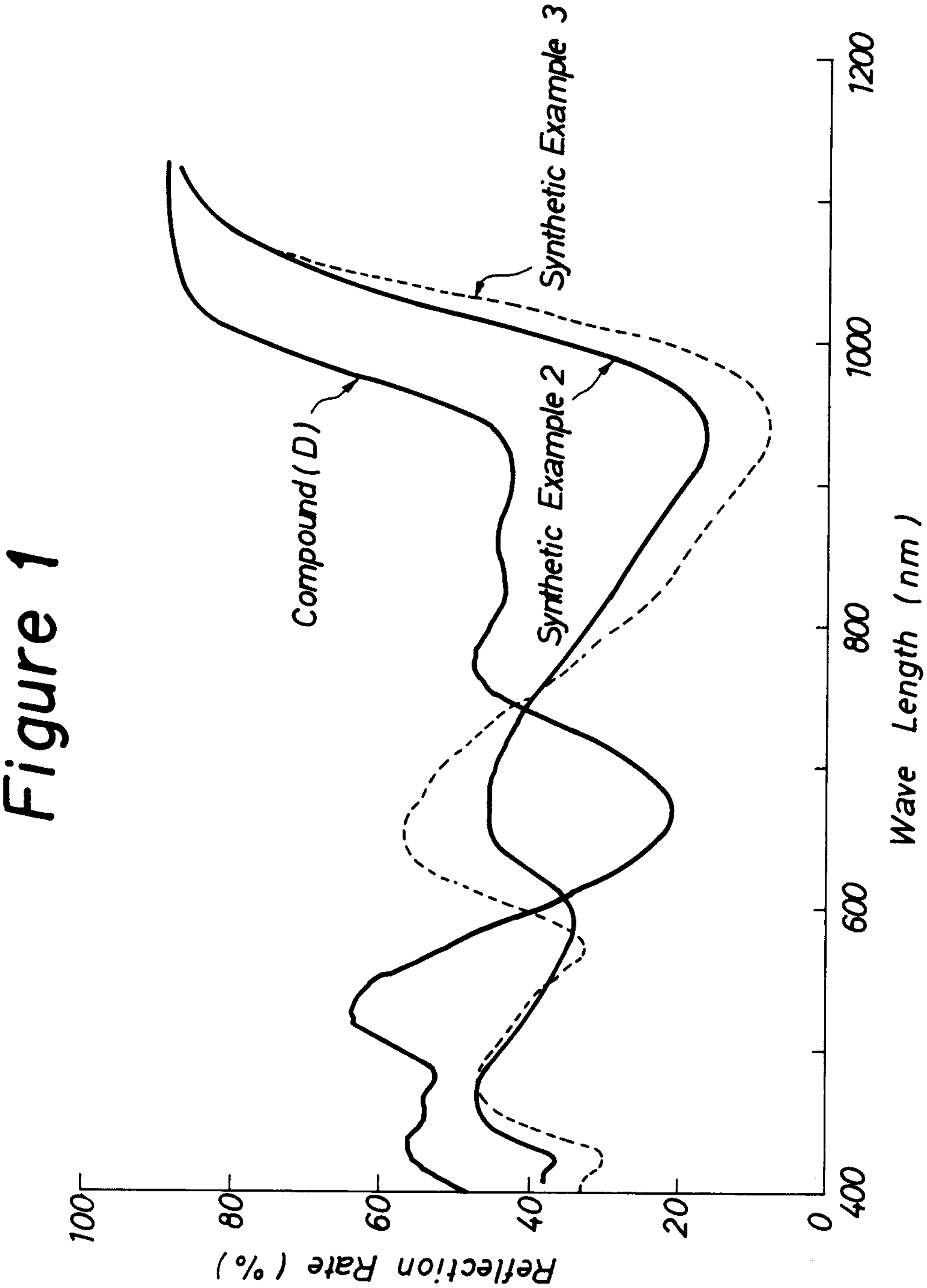
Synthetic Example 3
Compound (D)
Synthetic Example 2
Synthetic Example 1
Reflection Rate ( % )
100
80
60
40
20
0
400
600
800
1000
1200
Wave Length ( nm )

Figure2

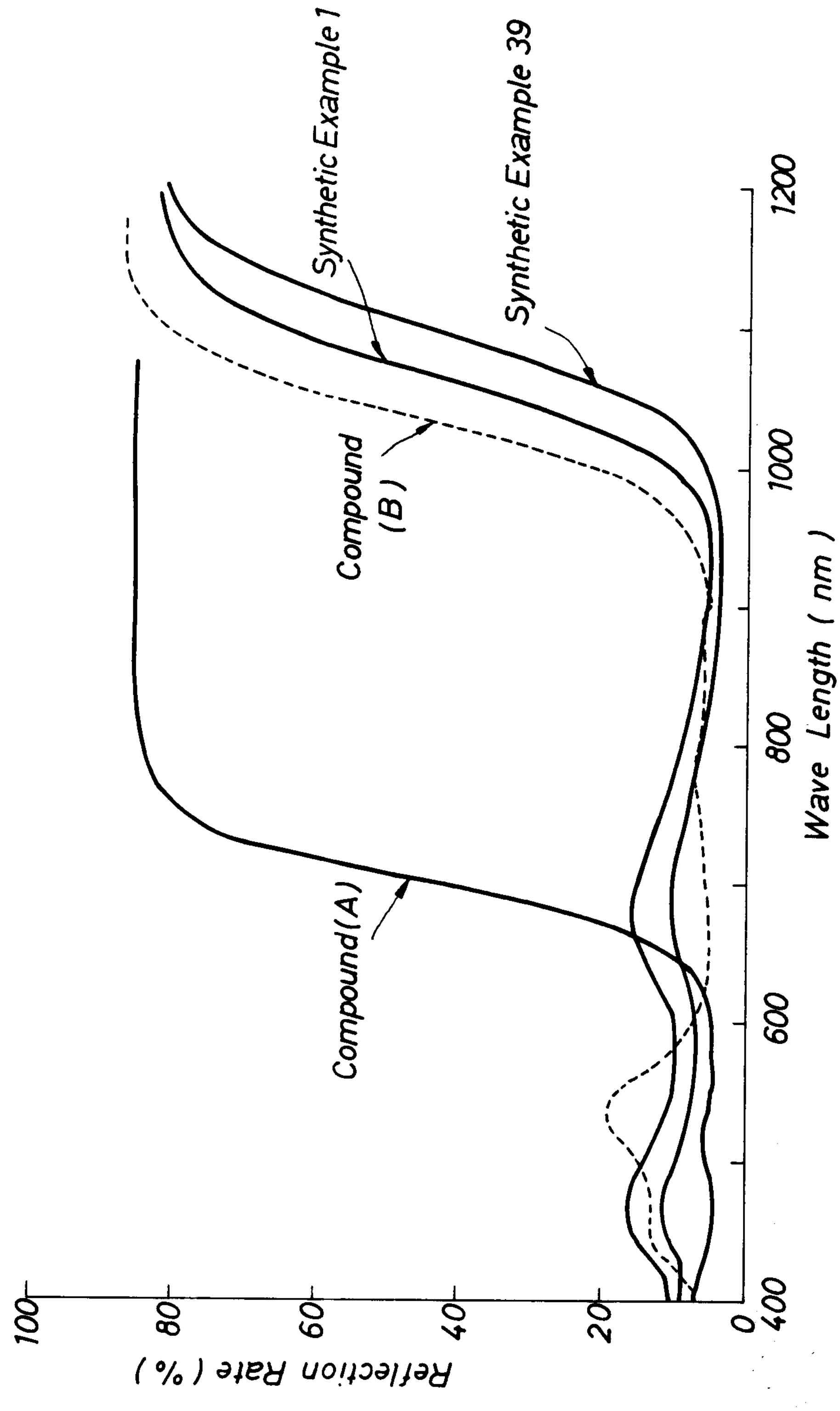
Reflection Rate ( % )
0
20
40
60
80
100
Wave Length ( nm )
400
600
800
1000
1200
Compound(A)
Compound
(B)
Synthetic Example 1
Synthetic Example 39

Figure 3

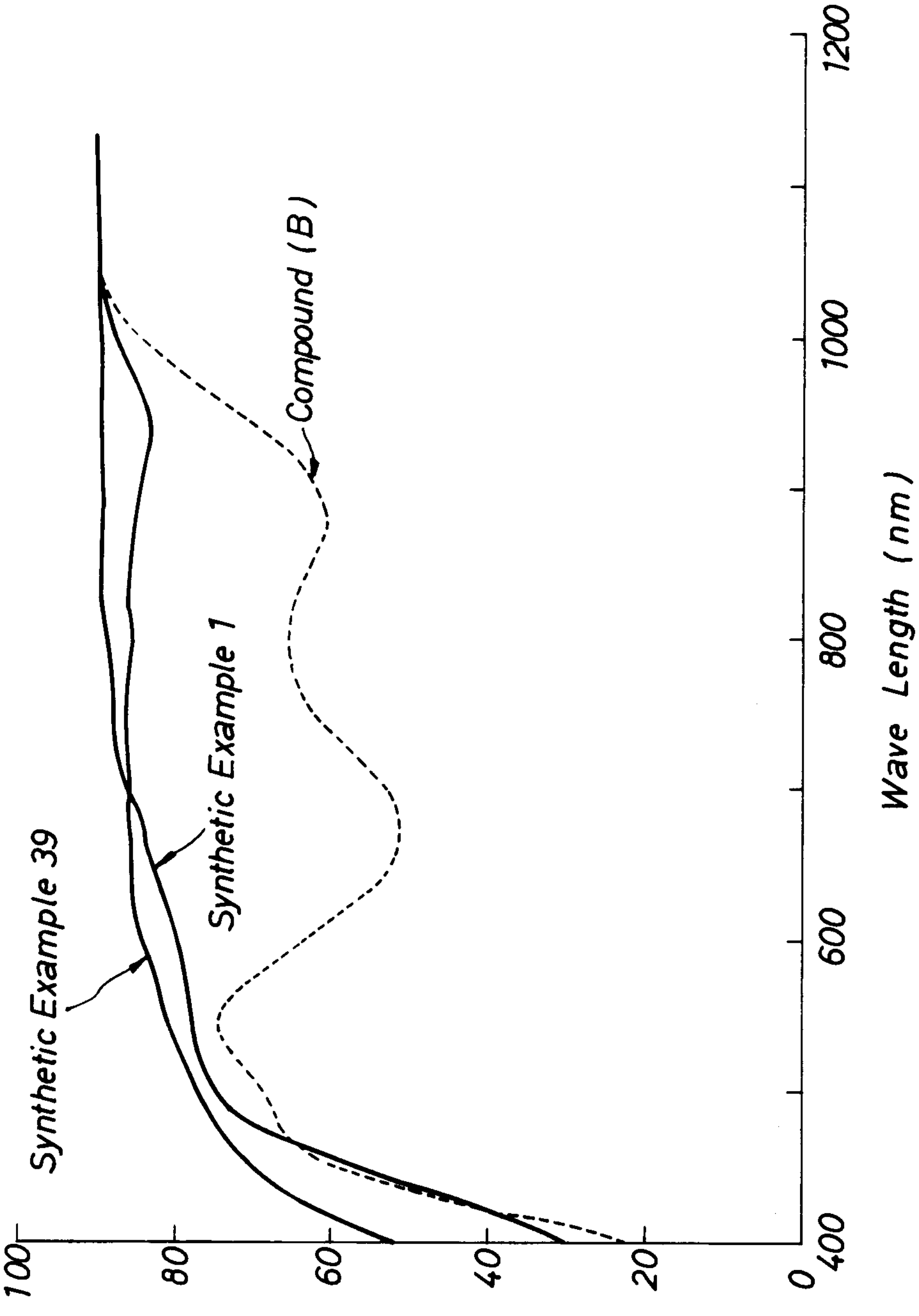
Synthetic Example 39
Synthetic Example 1
Compound (B)
Reflection Rate (%)
Wave Length (nm)
0
20
40
60
80
100
400
600
800
1000
1200